(19) Europäisches Patentamt
European Patent Office
Office européen des brevets

(11) EP 1 185 658 B1

(12) # EUROPEAN PATENT SPECIFICATION

(45) Date of publication and mention of the grant of the patent:
**04.06.2008 Bulletin 2008/23**

(21) Application number: **00927226.1**

(22) Date of filing: **18.05.2000**

(51) Int Cl.:
***C12N 15/31*** *(2006.01)* ***C07K 14/21*** *(2006.01)*
***C07K 16/12*** *(2006.01)* ***A61K 31/70*** *(2006.01)*
***A61K 39/02*** *(2006.01)* ***A61K 39/40*** *(2006.01)*
***G01N 33/569*** *(2006.01)*

(86) International application number:
**PCT/EP2000/004618**

(87) International publication number:
**WO 2000/071724 (30.11.2000 Gazette 2000/48)**

(54) **NOVEL COMPOUNDS FROM MORAXELLA CATARRHALIS**
NEUE VERBINDUNGEN AUS MORAXELLA CATARRHALIS
COMPOSES NOUVEAUX DE MORAXELLA CATARRHALIS

(84) Designated Contracting States:
**AT BE CH CY DE DK ES FI FR GB GR IE IT LI LU MC NL PT SE**

(30) Priority: **24.05.1999 GB 9912038**
**24.05.1999 GB 9912040**
**01.06.1999 GB 9912705**
**01.06.1999 GB 9912674**
**02.06.1999 GB 9912838**
**08.06.1999 GB 9913354**

(43) Date of publication of application:
**13.03.2002 Bulletin 2002/11**

(73) Proprietor: **GlaxoSmithKline Biologicals s.a.**
**1330 Rixensart (BE)**

(72) Inventor: **THONNARD, Joelle**
**SmithKline Beecham Bio. S.A.**
**B-1330 Rixensart (BE)**

(74) Representative: **Mallalieu, Catherine Louise**
**D Young & Co**
**120 Holborn**
**London EC1N 2DY (GB)**

(56) References cited:
**WO-A-95/31215**
**WO-A-97/32980**
**WO-A-97/41731**
**WO-A-98/06851**
**WO-A-98/28333**
**WO-A-98/33814**
**WO-A-99/58563**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 185 658 B1

SEQUENCE LISTING

<110> SmithKline Beecham Biologicals S.A.

<120> Novel Compounds

<130> BM45388

<160> 12

<170> FastSEQ for Windows Version 3.0

<210> 1
<211> 759
<212> DNA
<213> *Moraxella catarrhalis*

<400> 1

```
atgaaagctg ttaaattatc tgtagtttct gctgcagttc tactgtcaac ttctgcaatg    60
gcaaatgttg ttaccaatac tggtgcgacc gttgttgacg gtactcgtac catctttagt   120
acactggtta gacctgcggc tgtgagtgct gaggttggta ctttgggtta tggtgctaat   180
attggctggg cggtgaatga cactgttgag ctacaagcag gttgggctgg tggcaatatc   240
gcaaaatcca tcaatgataa ctttaatgcc aatggcgtta actatcagct tgaaactgat   300
ttttctaacc catacttagg tgctcaagtg cgtcctgctg ccaactggtt caccgttggg   360
acaggtatca tcgtacctaa gaacgacatc aaagtccgct caaataacac aaccgatggt   420
gttttccgtg ttgatggtaa agactacaaa caaagtgatg taggtcagct tactggtaag   480
cttgagcatc gtaacaaatt agcaccttat ttgacactgg gtttccgccc aaatctacac   540
agtaactggg gtgtatttgg tgaagttggt gctgcctatt tgggtaaagt agatgctact   600
gttgatgcac aaaatccaac caaatcagta actgctactg atagcaagac ccaagcgact   660
gtcattaaaa ttgcaaaaca agcagagcgt gacatcgaag ataaaaaata tgccaactgg   720
ttcccaatcg ctaaagttgg tgttacttac cgtttctaa                          759
```

<210> 2

<211> 252
<212> PRT
<213> *Moraxella catarrhalis*

<400> 2

```
Met Lys Ala Val Lys Leu Ser Val Val Ser Ala Ala Val Leu Leu Ser
 1               5                  10                  15
Thr Ser Ala Met Ala Asn Val Val Thr Asn Thr Gly Ala Thr Val Val
            20                  25                  30
Asp Gly Thr Arg Thr Ile Phe Ser Thr Leu Val Arg Pro Ala Ala Val
        35                  40                  45
Ser Ala Glu Val Gly Thr Leu Gly Tyr Gly Ala Asn Ile Gly Trp Ala
    50                  55                  60
Val Asn Asp Thr Val Glu Leu Gln Ala Gly Trp Ala Gly Gly Asn Ile
65                  70                  75                  80
Ala Lys Ser Ile Asn Asp Asn Phe Asn Ala Asn Gly Val Asn Tyr Gln
                85                  90                  95
Leu Glu Thr Asp Phe Ser Asn Pro Tyr Leu Gly Ala Gln Val Arg Pro
            100                 105                 110
Ala Ala Asn Trp Phe Thr Val Gly Thr Gly Ile Ile Val Pro Lys Asn
        115                 120                 125
Asp Ile Lys Val Arg Ser Asn Asn Thr Thr Asp Gly Val Phe Arg Val
    130                 135                 140
Asp Gly Lys Asp Tyr Lys Gln Ser Asp Val Gly Gln Leu Thr Gly Lys
145                 150                 155                 160
Leu Glu His Arg Asn Lys Leu Ala Pro Tyr Leu Thr Leu Gly Phe Arg
                165                 170                 175
Pro Asn Leu His Ser Asn Trp Gly Val Phe Gly Glu Val Gly Ala Ala
            180                 185                 190
Tyr Leu Gly Lys Val Asp Ala Thr Val Asp Ala Gln Asn Pro Thr Lys
        195                 200                 205
Ser Val Thr Ala Thr Asp Ser Lys Thr Gln Ala Thr Val Ile Lys Ile
    210                 215                 220
Ala Lys Gln Ala Glu Arg Asp Ile Glu Asp Lys Lys Tyr Ala Asn Trp
225                 230                 235                 240
Phe Pro Ile Ala Lys Val Gly Val Thr Tyr Arg Phe
                245                 250
```

<210> 3
<211> 1953
<212> DNA
<213> *Moraxella cacarrhalis*

<400> 3

```
atgattgatc atctttcttt taaaacgact aaggccaaaa gtcttcatac tcaaaatatc        60
gcccaaagaa cgctcaaagc ggtggctcat acataccgcc ctttatcaat ggcgattgca       120
ctgatgggct taagcagcat cgccaccgcc caagaattta gccaaaccgt attttttggt       180
gatagcctaa ctgacacagg acgcttggtg cagattggca aaaacagtct ggcgtcttct       240
gtttttaatc aagcccaacc atcgtttacg accaataccg atccagtttg gtcaagcata       300
ttggcaaaat cttatggaca taccgccgat gccaatgatg gaacaacctt aactggtaca       360
aactatgccg ttggtggtgc aagaactaaa gaagatgtgg tcaaaaatgc acctgttcct       420
tttttcacca ttcctttatt taccatccca tcagcacaaa cccaaatcaa tcgctacctg       480
accttaaata atcatcaagc cgaccccaaa gcattgtata ctgtttggac aggtgccaat       540
gatttgtttg aggcagccaa agcaccaacc cagttgcaag cagccgaaat cattaccacc       600
gctgccaatg accaagccaa tttggttggg cagcttgggc aagcaggtgc aaaacacatt       660
ttagtaccca gtcttcctga tgttggcgtc acgccagaat acgcccaaga tccgaccaaa       720
tctgccacag catcattgtc tgctcatatc tacaaccaaa ctttatatca aagtttaaac       780
aaccaaacca ccaatgtcat tgctgccaat acctttgccc tactcaaaga ggcggttagt       840
aatccagcag gatttgggtt tagtaatgtg accgaacctg cttgccaaaa cctagatgcc       900
aatttatcat cgttagcatg taaaccaagc gactggcaac aaaccgctgc gaatgctaat       960
gaaacttatg cgtttgctga taaaattcac ccttcagggc gtacgcatcg cattttggca      1020
caatattatc gctctattat cgatagcccc aaccaaatgg gtcaactacc ccagcatctg      1080
attaaacatg gtcaacaaag ccagcaacaa ttggtacgcc gcttagacag tcttaacaat      1140
catcaacaat cgctatggat tgatggcagc atcagcaatc aatcgcttga tacacgcaat      1200
aaaatagacc gccctaccat caaacttgga cttgatattg cacgacctaa ccagcataca      1260
ggggcatatt taacctacca acaacaagat tatcatctaa gcgattctat aaccgctgat      1320
gtcaaacaaa caggcattgg tttgtatcat cgccatgatt ttgatcactt acgcatcagt      1380
gccaaccttg ggggttgacca tttaagtaca caaagcttac gccaaatcat gtgggacggt      1440
gagaatcgca accatcaagc acatgccaca ggtaagcgtc gatatgcaag tgtacaaggc      1500
agctatggat ttacccaaaa caatgttacc tatcgtcctt atgtcggtat tcatgctcaa      1560
gatatcaagc agaatcgctt ctttgaaaat caaccaaatc tatcaactgc tctaagtttt      1620
aagctgcctg accatcagtc tttgcaagct gatattggcg ttaatattga ttatgccatg      1680
aatgataaac taaacctttt ggcaggattg ggttatcagc atgagtttaa agacaataac      1740
aaaactgttg agacagctgt attatccaac cgtgattatc atcgcagctt tgtcacaacc      1800
gtgccctttg ataaaaagca cacaacgcac gcacatttag gtgcaacact tgctttgggt      1860
aataacactc atttgaatgc tggcatacaa gccaatcacc aagatcatga tacaaaagtt      1920
ggtggatttg tgggtgcaca aatggcattt taa                                   1953
```

<210> 4
<211> 650
<212> PRT
<213> *Moraxella cacarrhalis*

<400> 4

```
Met Ile Asp His Leu Ser Phe Lys Thr Thr Lys Ala Lys Ser Leu His
 1               5                  10                  15
Thr Gln Asn Ile Ala Gln Arg Thr Leu Lys Ala Val Ala His Thr Tyr
            20                  25                  30
Arg Pro Leu Ser Met Ala Ile Ala Leu Met Gly Leu Ser Ser Ile Ala
        35                  40                  45
Thr Ala Gln Glu Phe Ser Gln Thr Val Phe Phe Gly Asp Ser Leu Thr
    50                  55                  60
Asp Thr Gly Arg Leu Val Gln Ile Gly Lys Asn Ser Leu Ala Ser Ser
65                  70                  75                  80
Val Phe Asn Gln Ala Gln Pro Ser Phe Thr Thr Asn Thr Asp Pro Val
                85                  90                  95
Trp Ser Ser Ile Leu Ala Lys Ser Tyr Gly His Thr Ala Asp Ala Asn
            100                 105                 110
Asp Gly Thr Thr Leu Thr Gly Thr Asn Tyr Ala Val Gly Gly Ala Arg
        115                 120                 125
Thr Lys Glu Asp Val Val Lys Asn Ala Pro Val Pro Phe Phe Thr Ile
    130                 135                 140
Pro Leu Phe Thr Ile Pro Ser Ala Gln Thr Gln Ile Asn Arg Tyr Leu
145                 150                 155                 160
Thr Leu Asn Asn His Gln Ala Asp Pro Lys Ala Leu Tyr Thr Val Trp
                165                 170                 175
Thr Gly Ala Asn Asp Leu Phe Glu Ala Ala Lys Ala Pro Thr Gln Leu
            180                 185                 190
Gln Ala Ala Glu Ile Ile Thr Thr Ala Ala Asn Asp Gln Ala Asn Leu
        195                 200                 205
Val Gly Gln Leu Gly Gln Ala Gly Ala Lys His Ile Leu Val Pro Ser
    210                 215                 220
Leu Pro Asp Val Gly Val Thr Pro Glu Tyr Ala Gln Asp Pro Thr Lys
225                 230                 235                 240
Ser Ala Thr Ala Ser Leu Ser Ala His Ile Tyr Asn Gln Thr Leu Tyr
                245                 250                 255
Gln Ser Leu Asn Asn Gln Thr Thr Asn Val Ile Ala Ala Asn Thr Phe
            260                 265                 270
Ala Leu Leu Lys Glu Ala Val Ser Asn Pro Ala Gly Phe Gly Phe Ser
        275                 280                 285
Asn Val Thr Glu Pro Ala Cys Gln Asn Leu Asp Ala Asn Leu Ser Ser
    290                 295                 300
Leu Ala Cys Lys Pro Ser Asp Trp Gln Gln Thr Ala Ala Asn Ala Asn
305                 310                 315                 320
Glu Thr Tyr Ala Phe Ala Asp Lys Ile His Pro Ser Gly Arg Thr His
                325                 330                 335
Arg Ile Leu Ala Gln Tyr Tyr Arg Ser Ile Ile Asp Ser Pro Asn Gln
            340                 345                 350
Met Gly Gln Leu Pro Gln His Leu Ile Lys His Gly Gln Gln Ser Gln
        355                 360                 365
Gln Gln Leu Val Arg Arg Leu Asp Ser Leu Asn Asn His Gln Gln Ser
    370                 375                 380
Leu Trp Ile Asp Gly Ser Ile Ser Asn Gln Ser Leu Asp Thr Arg Asn
385                 390                 395                 400
Lys Ile Asp Arg Pro Thr Ile Lys Leu Gly Leu Asp Ile Ala Arg Pro
                405                 410                 415
Asn Gln His Thr Gly Ala Tyr Leu Thr Tyr Gln Gln Gln Asp Tyr His
            420                 425                 430
```

```
Leu Ser Asp Ser Ile Thr Ala Asp Val Lys Gln Thr Gly Ile Gly Leu
        435                 440                 445
Tyr His Arg His Asp Phe Asp His Leu Arg Ile Ser Ala Asn Leu Gly
    450                 455                 460
Val Asp His Leu Ser Thr Gln Ser Leu Arg Gln Ile Met Trp Asp Gly
465                 470                 475                 480
Glu Asn Arg Asn His Gln Ala His Ala Thr Gly Lys Arg Arg Tyr Ala
                485                 490                 495
Ser Val Gln Gly Ser Tyr Gly Phe Thr Gln Asn Asn Val Thr Tyr Arg
            500                 505                 510
Pro Tyr Val Gly Ile His Ala Gln Asp Ile Lys Gln Asn Arg Phe Phe
        515                 520                 525
Glu Asn Gln Pro Asn Leu Ser Thr Ala Leu Ser Phe Lys Leu Pro Asp
    530                 535                 540
His Gln Ser Leu Gln Ala Asp Ile Gly Val Asn Ile Asp Tyr Ala Met
545                 550                 555                 560
Asn Asp Lys Leu Asn Leu Leu Ala Gly Leu Gly Tyr Gln His Glu Phe
                565                 570                 575
Lys Asp Asn Asn Lys Thr Val Glu Thr Ala Val Leu Ser Asn Arg Asp
            580                 585                 590
Tyr His Arg Ser Phe Val Thr Thr Val Pro Phe Asp Lys Lys His Thr
        595                 600                 605
Thr His Ala His Leu Gly Ala Thr Leu Ala Leu Gly Asn Asn Thr His
    610                 615                 620
Leu Asn Ala Gly Ile Gln Ala Asn His Gln Asp His Asp Thr Lys Val
625                 630                 635                 640
Gly Gly Phe Val Gly Ala Gln Met Ala Phe
                645                 650
```

<210> 5
<211> 1218
<212> DNA
<213> *Moraxella catarrhalis*

<400> 5

```
atgaaaaaaa cttccacaca gcttgggcta cttgccgtca gcgtttcgtt gattatggca      60
agtttacctg cacatgctgt ttatcttgac cgtaacttaa gagatggtct gaaatttggt     120
atcagtggtt ctgtcaatcc cagccttagc gtcaattcaa gcacttttac ttatttgggt     180
gactcatcag tatatggcaa taatgccact ttagagcgta tgctacaaga ccaagacaga     240
caagacagtg atgagcgagc aagactcaat ggatttggtg gtgcttctgt ttatctgggt     300
gcccaaaaat acctaactcg ggatattact ttatttggta atgttggttt gtatgcacca     360
gcaagcaaag gtcaaagagc tgcatatggc tatggcgtga atcttgccac caaatatggc     420
agtatcggta ttaatactga taatgaattt agtgctggtg ctggtacgcc cagcggaatt     480
tataatttgg ttgatggctc aaacgagtac agcactgcca tatcggttag taccagctat     540
atccctaagt ttaagtttag tgcataccat gcattgcctg gctcacctga tacacggtcg     600
gtaagtagcc acgaaaacta ctatatccaa aaagcacaag gtctttctgc atcttatagc     660
catcctatta gcccaaatca aaccctgtct gttggcacgg cttatagcaa aagccaaagg     720
cacaaagatt tttttagcga taccgcctat aataacaaaa cagcgtccac tgtggggcta     780
agttaccgcc aaggagattg gagtattagc ggtaatgttg gtcaagccaa agaaaattta     840
cacggtgcga tcattgatga tattaccaca aaagcttttg ggacaaaaat tagctacaaa     900
gtaaccccaa gaatttctgt ttctgggact tatggacaaa aaaccaccga taaaaatacc     960
aagcccaaca aacgcttgga tataccaaat atcatcgcac aacgaggagg taatatttca    1020
agccgtgtgc atgaaagttg gtttttttgat aaaaccaaac aaaaaaccta tggtctgagt    1080
gcaagttatt atatctatgg cggcatatcg atttctgctt cgatgaacca aacacgcacc    1140
accaacttta ccgaagaagg tgcttatagt gagcgtaaga ataacagcta tcgcatttca    1200
actggctttt cattttaa                                                  1218
```

<210> 6

<211> 405
<212> PRT
<213> *Moraxella catarrhalis*

<400> 6

```
Met Lys Lys Thr Ser Thr Gln Leu Gly Leu Leu Ala Val Ser Val Ser
 1               5                  10                  15
Leu Ile Met Ala Ser Leu Pro Ala His Ala Val Tyr Leu Asp Arg Asn
            20                  25                  30
Leu Arg Asp Gly Leu Lys Phe Gly Ile Ser Gly Ser Val Asn Pro Ser
        35                  40                  45
Leu Ser Val Asn Ser Ser Thr Phe Thr Tyr Leu Gly Asp Ser Ser Val
    50                  55                  60
Tyr Gly Asn Asn Ala Thr Leu Glu Arg Met Leu Gln Asp Gln Asp Arg
65                  70                  75                  80
Gln Asp Ser Asp Glu Arg Ala Arg Leu Asn Gly Phe Gly Gly Ala Ser
                85                  90                  95
Val Tyr Leu Gly Ala Gln Lys Tyr Leu Thr Arg Asp Ile Thr Leu Phe
            100                 105                 110
Gly Asn Val Gly Leu Tyr Ala Pro Ala Ser Lys Gly Gln Arg Ala Ala
        115                 120                 125
Tyr Gly Tyr Gly Val Asn Leu Ala Thr Lys Tyr Gly Ser Ile Gly Ile
    130                 135                 140
Asn Thr Asp Asn Glu Phe Ser Ala Gly Ala Gly Thr Pro Ser Gly Ile
145                 150                 155                 160
Tyr Asn Leu Val Asp Gly Ser Asn Glu Tyr Ser Thr Ala Ile Ser Val
                165                 170                 175
Ser Thr Ser Tyr Ile Pro Lys Phe Lys Phe Ser Ala Tyr His Ala Leu
            180                 185                 190
Pro Gly Ser Pro Asp Thr Arg Ser Val Ser Ser His Glu Asn Tyr Tyr
        195                 200                 205
Ile Gln Lys Ala Gln Gly Leu Ser Ala Ser Tyr Ser His Pro Ile Ser
    210                 215                 220
Pro Asn Gln Thr Leu Ser Val Gly Thr Ala Tyr Ser Lys Ser Gln Arg
225                 230                 235                 240
His Lys Asp Phe Phe Ser Asp Thr Ala Tyr Asn Asn Lys Thr Ala Ser
                245                 250                 255
Thr Val Gly Leu Ser Tyr Arg Gln Gly Asp Trp Ser Ile Ser Gly Asn
            260                 265                 270
Val Gly Gln Ala Lys Glu Asn Leu His Gly Ala Ile Ile Asp Asp Ile
        275                 280                 285
Thr Thr Lys Ala Phe Gly Thr Lys Ile Ser Tyr Lys Val Thr Pro Arg
    290                 295                 300
Ile Ser Val Ser Gly Thr Tyr Gly Gln Lys Thr Thr Asp Lys Asn Thr
305                 310                 315                 320
Lys Pro Asn Lys Arg Leu Asp Ile Pro Asn Ile Ile Ala Gln Arg Gly
                325                 330                 335
Gly Asn Ile Ser Ser Arg Val His Glu Ser Trp Phe Phe Asp Lys Thr
            340                 345                 350
Lys Gln Lys Thr Tyr Gly Leu Ser Ala Ser Tyr Tyr Ile Tyr Gly Gly
        355                 360                 365
Ile Ser Ile Ser Ala Ser Met Asn Gln Thr Arg Thr Thr Asn Phe Thr
    370                 375                 380
Glu Glu Gly Ala Tyr Ser Glu Arg Lys Asn Asn Ser Tyr Arg Ile Ser
385                 390                 395                 400
Thr Gly Phe Ser Phe
                405
```

<210> 7
<211> 1233

<212> DNA
<213> *Moraxella catarrhalis*

<400> 7

```
atgaaaccat caatcatcaa aaacccatta aagcttttga ccgccatgat tttgttaaac      60
gcaatgccta gccatgcgat ttataatctg tataaaagcg gtgattttag cttcgatgtg     120
catggtgaga ttaacactta tggcaaaaa aatagccaaa aatataccta cctttatcca     180
gaaacaggct gggcaagtgt taacaatgac tacgaagcca tcaccaaagg tgcttatgag     240
caaagaagtg atcgccgtgt acgcttgggg caagatacag gtgcttcttg gacggagttt     300
cgtgcatcta gaaaactaag agatggctgg cgtgtcagtg ggcaattgg ttttggttat     360
tatgatagcg gtacaggtat gtacctaaat agtgctaata tggcatttga taaaaaagat     420
ttaggttctt tatcattggg tcgtcaatat ctgcacacag gctatgtcac acgcaccaat     480
acctatacac cacttgaaac ttttggggaa aatagcatcc gcttggatta tactgccatt     540
aaaggattgc atgccagtgg ctattatagc ctgccagctt caagcgatgt tcgtaaaaag     600
aagaatgggc aagaggtaga gggtttggt gcgtctgtta gctaccttca tccacttgat     660
gatcatcaaa cggtgcgtgt tgcattgggc tatagtgata gccgtcaaaa cgcaaaaacc     720
acaggtagag acagtaattt ttatgctacc aagagccaag gtacggcagc ttcggttgaa     780
tatcgcaaca ataaactgct attggcggca gatattgggc aaaaaaatga acaaatcaat     840
ggtactgtta ccgataaatc taagggcaac tacatgggcg ttaaggtagg ttatgaaatc     900
acgcctcgcc taaccatgac ggcaggcttt ggtaaaaaag ttgctgaacg cacccaaaaa     960
agcggtgcag cactgataca tgcctaccaa aaagacttgt gtgttgctga tgccaatgat    1020
tcatgccata attttgttca ggcttatgaa acagcgttat ttgataagat tgattcaaaa    1080
cgctcttatg tgcgtgccga ttattatcta agagaaaatg tacgcctata cggccgtatt    1140
gatgacgaaa aaattaccaa tcagttgggt aataaagact ttagcaagct tcacaataca    1200
ggctatcgtg ctggcgtgtc attcatttc taa                                  1233
```

<210> 8
<211> 410
<212> PRT
<213> *Moraxella catarrhalis*

<400> 8

```
Met Lys Pro Ser Ile Ile Lys Asn Pro Leu Lys Leu Leu Thr Ala Met
 1               5                  10                  15
Ile Leu Leu Asn Ala Met Pro Ser His Ala Ile Tyr Asn Leu Tyr Lys
            20                  25                  30
Ser Gly Asp Phe Ser Phe Asp Val His Gly Glu Ile Asn Thr Tyr Gly
        35                  40                  45
Gln Lys Asn Ser Gln Lys Tyr Thr Tyr Leu Tyr Pro Glu Thr Gly Trp
    50                  55                  60
Ala Ser Val Asn Asn Asp Tyr Glu Ala Ile Thr Lys Gly Ala Tyr Glu
65                  70                  75                  80
Gln Arg Ser Asp Arg Arg Val Arg Leu Gly Gln Asp Thr Gly Ala Ser
                85                  90                  95
Trp Thr Glu Phe Arg Ala Ser Arg Lys Leu Arg Asp Gly Trp Arg Val
            100                 105                 110
Ser Gly Ala Ile Gly Phe Gly Tyr Tyr Asp Ser Gly Thr Gly Met Tyr
        115                 120                 125
Leu Asn Ser Ala Asn Met Ala Phe Asp Lys Lys Asp Leu Gly Ser Leu
    130                 135                 140
Ser Leu Gly Arg Gln Tyr Leu His Thr Gly Tyr Val Thr Arg Thr Asn
145                 150                 155                 160
Thr Tyr Thr Pro Leu Glu Thr Phe Gly Glu Asn Ser Ile Arg Leu Asp
                165                 170                 175
Tyr Thr Ala Ile Lys Gly Leu His Ala Ser Gly Tyr Tyr Ser Leu Pro
            180                 185                 190
```

```
Ala Ser Ser Asp Val Arg Lys Lys Lys Asn Gly Gln Glu Val Glu Gly
        195                 200                 205
Phe Gly Ala Ser Val Ser Tyr Leu His Pro Leu Asp Asp His Gln Thr
    210                 215                 220
Val Arg Val Ala Leu Gly Tyr Ser Asp Ser Arg Gln Asn Ala Lys Thr
225                 230                 235                 240
Thr Gly Arg Asp Ser Asn Phe Tyr Ala Thr Lys Ser Gln Gly Thr Ala
                245                 250                 255
Ala Ser Val Glu Tyr Arg Asn Asn Lys Leu Leu Leu Ala Ala Asp Ile
            260                 265                 270
Gly Gln Lys Asn Glu Gln Ile Asn Gly Thr Val Thr Asp Lys Ser Lys
        275                 280                 285
Gly Asn Tyr Met Gly Val Lys Val Gly Tyr Glu Ile Thr Pro Arg Leu
    290                 295                 300
Thr Met Thr Ala Gly Phe Gly Lys Lys Val Ala Glu Arg Thr Gln Lys
305                 310                 315                 320
Ser Gly Ala Ala Leu Ile His Ala Tyr Gln Lys Asp Leu Cys Val Ala
                325                 330                 335
Asp Ala Asn Asp Ser Cys His Asn Phe Val Gln Ala Tyr Glu Thr Ala
            340                 345                 350
Leu Phe Asp Lys Ile Asp Ser Lys Arg Ser Tyr Val Arg Ala Asp Tyr
        355                 360                 365
Tyr Leu Arg Glu Asn Val Arg Leu Tyr Gly Arg Ile Asp Asp Glu Lys
    370                 375                 380
Ile Thr Asn Gln Leu Gly Asn Lys Asp Phe Ser Lys Leu His Asn Thr
385                 390                 395                 400
Gly Tyr Arg Ala Gly Val Ser Phe Ile Phe
                405                 410
```

<210> 9
<211> 2457
<212> DNA
<213> *Moraxella catarrhalis*

<400> 9

```
atgaaggtta ccatgataaa aaaaccgctt gcctgtgcca tattggcaac tttttcaatg      60
ccaatgctgg cagaggcgaa tttaaaggat aagccaaccg tcattttaga tggcgtttcg     120
atcacctctt tagctgacca aaatacagag tttggcgtta atcattcaaa aacagtcagt     180
ggcatcacag tttcaaaaga gcaactacaa caacgagcaa ccaccctagg cgatgccttg     240
gcaggtgagc ttggcgttca ttctaaccat tttgggggcg gtgcctcagc cccccatcatt    300
cgtgggcagg agggtaaacg cctgaaaatc ctacaaaacg gttcagaggt tgtggacatg     360
tctgggttgt cgccagacca tgccatagcg gtggacacca cactggcaaa acaggtggag     420
attgtgcgag gctctggtgc cttgttgtac gcctctggca actcagcagg cgtggtcaat     480
gtcgttgatg acaaaatacc cagcaaattg cccagcaaat tacaaggtga tgtgacggtg     540
cgtcttagca gtgccaaccg tgaaaaatta atcaccgcca gtgccgaagc cccactggga     600
gagcatgtgg cagtgcgtgt tgcagggctg tccaaacaag cagcagacta taaaacgcca     660
cgctttgacc gccatgtctt taacaaaaaa catgaagatg ataacactca gccagaattc     720
atctataaag acaccttaaa gcatctgcca gacagccatg ccaaatcaaa cgcaggaacg     780
cttggcgtgt catgggttgg caatcaaggc tttttggggg catcggtgag cttacgccga     840
gacaaatatg gcctgcccaa ccattcacat gaatatgaag aatgtagcgt gcatggcatt     900
tctcagtccg ccttacaata caagccatat ttgcgtttgt atcctttttt gatggaaaat     960
gatgacttag agtttgacaa tgcagggctt gaatgccata cacatgatga ccacgaccac    1020
gagcacgacc atgctcatga ccacgagcac gaccacgagc acgaccatgg caaaccttgg    1080
attgatttga aaatgaagcg ttatgatgtg caagggcaaa tcaatgcgcc gtttgctggc    1140
attgataaaa tccgagccag catgggcaaa gtggattatc atcatgatga gatagatggg    1200
ggtgagaaga ccagcttttt tgataatcaa gccaatgtgt ggcgtctgga agcctcacat    1260
accccccattc atacgccgat gggcaagttt agcggggtgt ttggggtagg ttatctcacc   1320
tcaaaaaaca gcggacttgt gccacctcgt tatgaagatg gcaataaaca agacacccaa    1380
```

```
aacatcttgc acaataataa aaccaaaaca ggcagtgtgt tttggtttga agaatacaaa    1440
cccaatgaca agctgaccgt tgacgccgcc gctcgcattg agaaacaaac catcaccatg    1500
gattatgata aagacgccat ttatcagagc ttaaacttag gcttagcaac cgctcatgaa    1560
ccagacatac gctttaaacg attgctggac agcggtactt taaaccccaa aaaacaaacc    1620
gcacgctctt atgctgttgg gacgcattta caattaacgc ccaaacataa attatcgctg    1680
aatctgtcgc atcaagaacg cctgccaaat gctcaggaat tgtatgctca cggcatgcac    1740
cttgccacca actcgtttga aattggcaac cgctttttaa acaaagaaaa atccaacaac    1800
attgatttgg ggctgacatt tcaaggtgat aaatgggatt atcgtcttgg gggctatcat    1860
tatgattttg ataactatgt gtttttacaa acattgtcgc agtataagca aggtttgcgt    1920
ggcatgcgtc atgataaaga cttaaaaacc gcacgctatg aacaagcagc ggcgaaattt    1980
tatggatttg atgtcaacat cggttatcag attaatgatg tatatcatgt ggcgttattt    2040
ggtgattata ttcgtggcaa gctcaccaat ttgcctgaca aaaagggcag aaccgatgcg    2100
tatggcaacc gtcctctcat caaacagcca gacagtcata cgccaagact gccaccaaaa    2160
cgccttggca tgaaattaac cgccaatgtt aatgcaaatt ggtcagggtt tttggaatat    2220
cgccatacct ttaaacaaga taaattggcg aattttgaac gcccaacccc agctcataac    2280
ttggtgaatt tggggcttaa ctatcagcac aagccaagcc atcaagcagg ctcggttcag    2340
gtatttttta atgctaacaa tctattaaac gataaagtct ttgctcatga gacatttttc    2400
ccagacatgc cacaaatggg gcgaaacttt atgctcgggg caaatttcaa attttga       2457
```

<210> 10
<211> 818
<212> PRT
<213> *Moraxelia catarrhalis*

<400> 10

```
Met Lys Val Thr Met Ile Lys Lys Pro Leu Ala Cys Ala Ile Leu Ala
 1               5                  10                  15
Thr Phe Ser Met Pro Met Leu Ala Glu Ala Asn Leu Lys Asp Lys Pro
            20                  25                  30
Thr Val Ile Leu Asp Gly Val Ser Ile Thr Ser Leu Ala Asp Gln Asn
        35                  40                  45
Thr Glu Phe Gly Val Asn His Ser Lys Thr Val Ser Gly Ile Thr Val
    50                  55                  60
Ser Lys Glu Gln Leu Gln Gln Arg Ala Thr Thr Leu Gly Asp Ala Leu
65                  70                  75                  80
Ala Gly Glu Leu Gly Val His Ser Asn His Phe Gly Gly Gly Ala Ser
                85                  90                  95
Ala Pro Ile Ile Arg Gly Gln Glu Gly Lys Arg Leu Lys Ile Leu Gln
            100                 105                 110
Asn Gly Ser Glu Val Val Asp Met Ser Gly Leu Ser Pro Asp His Ala
        115                 120                 125
Ile Ala Val Asp Thr Thr Leu Ala Lys Gln Val Glu Ile Val Arg Gly
    130                 135                 140
Ser Gly Ala Leu Leu Tyr Ala Ser Gly Asn Ser Ala Gly Val Val Asn
145                 150                 155                 160
Val Val Asp Asp Lys Ile Pro Ser Lys Leu Pro Ser Lys Leu Gln Gly
                165                 170                 175
Asp Val Thr Val Arg Leu Ser Ser Ala Asn Arg Glu Lys Leu Ile Thr
            180                 185                 190
Ala Ser Ala Glu Ala Pro Leu Gly Glu His Val Ala Val Arg Val Ala
        195                 200                 205
Gly Leu Ser Lys Gln Ala Ala Asp Tyr Lys Thr Pro Arg Phe Asp Arg
    210                 215                 220
His Val Phe Asn Lys Lys His Glu Asp Asp Asn Thr Gln Pro Glu Phe
225                 230                 235                 240
Ile Tyr Lys Asp Thr Leu Lys His Leu Pro Asp Ser His Ala Lys Ser
                245                 250                 255
Asn Ala Gly Thr Leu Gly Val Ser Trp Val Gly Asn Gln Gly Phe Leu
```

```
            260                 265                 270
Gly Ala Ser Val Ser Leu Arg Arg Asp Lys Tyr Gly Leu Pro Asn His
        275                 280                 285
Ser His Glu Tyr Glu Glu Cys Ser Val His Gly Ile Ser Gln Ser Ala
    290                 295                 300
Leu Gln Tyr Lys Pro Tyr Leu Arg Leu Tyr Pro Phe Leu Met Glu Asn
305                 310                 315                 320
Asp Asp Leu Glu Phe Asp Asn Ala Gly Leu Glu Cys His Thr His Asp
                325                 330                 335
Asp His Asp His Glu His Asp His Ala His Asp His Glu His Asp His
            340                 345                 350
Glu His Asp His Gly Lys Pro Trp Ile Asp Leu Lys Met Lys Arg Tyr
        355                 360                 365
Asp Val Gln Gly Gln Ile Asn Ala Pro Phe Ala Gly Ile Asp Lys Ile
    370                 375                 380
Arg Ala Ser Met Gly Lys Val Asp Tyr His His Asp Glu Ile Asp Gly
385                 390                 395                 400
Gly Glu Lys Thr Ser Phe Phe Asp Asn Gln Ala Asn Val Trp Arg Leu
                405                 410                 415
Glu Ala Ser His Thr Pro Ile His Thr Pro Met Gly Lys Phe Ser Gly
            420                 425                 430
Val Phe Gly Val Gly Tyr Leu Thr Ser Lys Asn Ser Gly Leu Val Pro
        435                 440                 445
Pro Arg Tyr Glu Asp Gly Asn Lys Gln Asp Thr Gln Asn Ile Leu His
    450                 455                 460
Asn Asn Lys Thr Lys Thr Gly Ser Val Phe Trp Phe Glu Glu Tyr Lys
465                 470                 475                 480
Pro Asn Asp Lys Leu Thr Val Asp Ala Ala Ala Arg Ile Glu Lys Gln
                485                 490                 495
Thr Ile Thr Met Asp Tyr Asp Lys Asp Ala Ile Tyr Gln Ser Leu Asn
            500                 505                 510
Leu Gly Leu Ala Thr Ala His Glu Pro Asp Ile Arg Phe Lys Arg Leu
        515                 520                 525
Leu Asp Ser Gly Thr Leu Asn Pro Lys Lys Gln Thr Ala Arg Ser Tyr
    530                 535                 540
Ala Val Gly Thr His Leu Gln Leu Thr Pro Lys His Lys Leu Ser Leu
545                 550                 555                 560
Asn Leu Ser His Gln Glu Arg Leu Pro Asn Ala Gln Glu Leu Tyr Ala
                565                 570                 575
His Gly Met His Leu Ala Thr Asn Ser Phe Glu Ile Gly Asn Arg Phe
            580                 585                 590
Leu Asn Lys Glu Lys Ser Asn Asn Ile Asp Leu Gly Leu Thr Phe Gln
        595                 600                 605
Gly Asp Lys Trp Asp Tyr Arg Leu Gly Gly Tyr His Tyr Asp Phe Asp
    610                 615                 620
Asn Tyr Val Phe Leu Gln Thr Leu Ser Gln Tyr Lys Gln Gly Leu Arg
625                 630                 635                 640
Gly Met Arg His Asp Lys Asp Leu Lys Thr Ala Arg Tyr Glu Gln Ala
                645                 650                 655
Ala Ala Lys Phe Tyr Gly Phe Asp Val Asn Ile Gly Tyr Gln Ile Asn
            660                 665                 670
Asp Val Tyr His Val Ala Leu Phe Gly Asp Tyr Ile Arg Gly Lys Leu
        675                 680                 685
Thr Asn Leu Pro Asp Lys Lys Gly Arg Thr Asp Ala Tyr Gly Asn Arg
    690                 695                 700
Pro Leu Ile Lys Gln Pro Asp Ser His Thr Pro Arg Leu Pro Pro Lys
705                 710                 715                 720
Arg Leu Gly Met Lys Leu Thr Ala Asn Val Asn Ala Asn Trp Ser Gly
```

```
                        725                 730                 735
Phe Leu Glu Tyr Arg His Thr Phe Lys Gln Asp Lys Leu Ala Asn Phe
                    740                 745                 750
Glu Arg Pro Thr Pro Ala His Asn Leu Val Asn Leu Gly Leu Asn Tyr
                755                 760                 765
Gln His Lys Pro Ser His Gln Ala Gly Ser Val Gln Val Phe Phe Asn
            770                 775                 780
Ala Asn Asn Leu Leu Asn Asp Lys Val Phe Ala His Glu Thr Phe Phe
785                 790                 795                 800
Pro Asp Met Pro Gln Met Gly Arg Asn Phe Met Leu Gly Ala Asn Phe
                805                 810                 815
Lys Phe
```

<210> 11
<211> 2742
<212> DNA
<213> *Moraxella cacarrhalis*

<400> 11

```
atgataaaaa aaccacttgt ttgtgcgata tcggccacct ttgcgatgcc agcggtagca     60
gataatacca agctgggtga agagccaacc accaccttaa agggtgtatt ggtaagctcg    120
caaacgaacc aaaatacagg ttttgtatct aatgattcaa aacaatccag tgatcttacg    180
ctttcaaaag ataaattaaa atatcgttcg gcaaccttgg gcaatgcgtt aagtggtgag    240
cttggtattc atagtaaccc ttttggtggc ggttcatctg cacctgttgt gcgagggcaa    300
gagggtgtgc gtcttaagat tttacaaaat ggaactgatg tgattgatgt gtcatcaata    360
tcgcctgatc atgttgtggc gaccgataca cttttagcgt ctaaagttga gcttgttcgt    420
ggtgctgata cgctgttata tggcttggca tcgccagctg gtgtgattaa tgttgttgat    480
gaccgtatcc cgaatcgtat gcctagtggt gctatccatg acaaaatcga aggcgagacg    540
atgcttcgat ataacacaaa caaccatgaa aagcttgcaa ctgcaggggt gagctttggg    600
gtaggagatc gcattgcggt tcgggtggag ggcttaaagc gagaggctga tgactatcaa    660
gttccccatt ttcaggcaga tcgcatgtta gattatgtgc caggtagtgc aaataactct    720
accgttggca tgattggcgt gtcttatatt catgataatg ggcatatcgg tgcttcttat    780
agccaccgta aagatcgtta tggtatccca ggcatatcc actgcgacag ccaacgagag     840
cattttatca aatggcataa tatcacaaaa tccaattatt atttacccat ttatcctcat    900
ttgatggagg attcagatat tgatgacaat cctcatacgc attgccgcca caaccacgaa    960
gaccatatcg gtgagcataa tcccacgggc gtgcccatca atcatgaaca tcattcgcct   1020
tggattgata tgaaaaccaa tcgctacgac attcgtggcg aggtatatcg gcctattcaa   1080
ggtttggata aaattaagct aagcttaact tatgcagatt attatcatga tgaaaaagat   1140
gctggcaatg agcaagaccc aaacaatcac aaaccttctg agcgtgatac aacggtggat   1200
aagggtcatg ccagctctat ttttacaaaa aaaggcgtta atggtcgctt ggagttatat   1260
catacaccga ccaaacgctt atctggggta ttgggtattg ggtatcaaac ccaaaaatct   1320
gcagcaggag aggcgtattt gccaagctat tttcaatcag aagcagaatg gcaaaaagcc   1380
caaagtcaaa acattaacca atatcgtcct tacttattag ttccaaatac caataaaagc   1440
cttggtattt ttggacttga gcaactaaag ctaaatcaaa tgacttttaa ggtggcgatg   1500
cgtcatgaaa gacaaaaaac accaattgaa tatgaccagc atttacttga ccatgctttg   1560
cagtattttt taagtaaagc acagctaaaa gcacctgatc atcctgattt gacgacatat   1620
aaacaacatg ccacctctta tgctggtagt gccttatggg atattacgcc aaatcatcga   1680
ttgtcattga cctactcaca taacgaacgc attccatcgc cgatggagct gtattatcaa   1740
ggcggacatt tggcgaccag ctcttttgag catggcaata aaaacttggt caaagaaaaa   1800
tcggataatt atgagctggg ttttatgcat acagcagata aagtcagcta taaagcaagc   1860
acttactata gcaattttga taactatatt tttaatgaga ccattgccaa agaaggcaat   1920
ttatacatca gacgctataa tcagacgacg gctaagtttt atggtgtgga gggttcatta   1980
acttaccagc caaatgccaa tcacagtgtg atgttttttg gtgatatggt gcaaggtaaa   2040
attggtgcat tatctgatat taaaggtaag cttgtatatg ctggcagaaa gtgggtttat   2100
tttgatgatg atatcaagga tatgaccgtt gatgataatg gtgattatga tgccgatggt   2160
ggtttgactt gtgccttaaa aacgcctgaa caatggggac aaattaacga taataatgat   2220
tgtagtacaa ccattaatgt ctataaaaac ggtacaacaa cctcaggcga ggaggattac   2280
```

```
gaccgtttgg cacgaaatcc aacttacgca cccagagtac cgcccagccg cttgggcatt    2340
cgttggcaag ggtattttgg tgatcattgg tctgccaatg cagaatttaa ccatgtgttt    2400
gcacaaaata aagttgccac ctcaacggtt gccattaaac ctcaattcaa gcagccagaa    2460
ggttgccaac gccatgagag tcattgccga atcagcgact atggcagtga taacaacccct   2520
ttgatgatgc agccaagata tatcacagaa aacaaaacgg caggatataa tttgctcaat    2580
gttggcttag attataacaa tgcatatcgt aatgttgatt atacgctgtc aattcgtgcg    2640
aataatttac ttaatgaaca aatctacatt cacaactcat ttttgccgtt tgtaccgcag    2700
atggggcgta atctgacttt aggtttgacg gctaaatttt ga                        2742
```

<210> 12
<211> 913
<212> PRT
<213> *Moraxella catarrhalis*

<400> 12

```
Met Ile Lys Lys Pro Leu Val Cys Ala Ile Ser Ala Thr Phe Ala Met
 1               5                  10                  15
Pro Ala Val Ala Asp Asn Thr Lys Leu Gly Glu Glu Pro Thr Thr Thr
            20                  25                  30
Leu Lys Gly Val Leu Val Ser Ser Gln Thr Asn Gln Asn Thr Gly Phe
        35                  40                  45
Val Ser Asn Asp Ser Lys Gln Ser Ser Asp Leu Thr Leu Ser Lys Asp
    50                  55                  60
Lys Leu Lys Tyr Arg Ser Ala Thr Leu Gly Asn Ala Leu Ser Gly Glu
65                  70                  75                  80
Leu Gly Ile His Ser Asn Pro Phe Gly Gly Gly Ser Ser Ala Pro Val
                85                  90                  95
Val Arg Gly Gln Glu Gly Val Arg Leu Lys Ile Leu Gln Asn Gly Thr
            100                 105                 110
Asp Val Ile Asp Val Ser Ser Ile Ser Pro Asp His Val Val Ala Thr
        115                 120                 125
Asp Thr Leu Leu Ala Ser Lys Val Glu Leu Val Arg Gly Ala Asp Thr
    130                 135                 140
Leu Leu Tyr Gly Leu Ala Ser Pro Ala Gly Val Ile Asn Val Val Asp
145                 150                 155                 160
Asp Arg Ile Pro Asn Arg Met Pro Ser Gly Ala Ile His Asp Lys Ile
                165                 170                 175
Glu Gly Glu Thr Met Leu Arg Tyr Asn Thr Asn Asn His Glu Lys Leu
            180                 185                 190
Ala Thr Ala Gly Val Ser Phe Gly Val Gly Asp Arg Ile Ala Val Arg
        195                 200                 205
Val Glu Gly Leu Lys Arg Glu Ala Asp Asp Tyr Gln Val Pro His Phe
    210                 215                 220
Gln Ala Asp Arg Met Leu Asp Tyr Val Pro Gly Ser Ala Asn Asn Ser
225                 230                 235                 240
Thr Val Gly Met Ile Gly Val Ser Tyr Ile His Asp Asn Gly His Ile
                245                 250                 255
Gly Ala Ser Tyr Ser His Arg Lys Asp Arg Tyr Gly Ile Pro Gly His
            260                 265                 270
Ile His Cys Asp Ser Gln Arg Glu His Phe Ile Lys Trp His Asn Ile
        275                 280                 285
Thr Lys Ser Asn Tyr Tyr Leu Pro Ile Tyr Pro His Leu Met Glu Asp
    290                 295                 300
Ser Asp Ile Asp Asp Asn Pro His Thr His Cys Arg His Asn His Glu
305                 310                 315                 320
Asp His Ile Gly Glu His Asn Pro Thr Gly Val Pro Ile Asn His Glu
                325                 330                 335
His His Ser Pro Trp Ile Asp Met Lys Thr Asn Arg Tyr Asp Ile Arg
```

```
            340                 345                 350
Gly Glu Val Tyr Arg Pro Ile Gln Gly Leu Asp Lys Ile Lys Leu Ser
        355                 360                 365
Leu Thr Tyr Ala Asp Tyr Tyr His Asp Glu Lys Asp Ala Gly Asn Glu
    370                 375                 380
Gln Asp Pro Asn Asn His Lys Pro Ser Glu Arg Asp Thr Thr Val Asp
385                 390                 395                 400
Lys Gly His Ala Ser Ser Ile Phe Thr Lys Lys Gly Val Asn Gly Arg
                405                 410                 415
Leu Glu Leu Tyr His Thr Pro Thr Lys Arg Leu Ser Gly Val Leu Gly
            420                 425                 430
Ile Gly Tyr Gln Thr Gln Lys Ser Ala Ala Gly Glu Ala Tyr Leu Pro
        435                 440                 445
Ser Tyr Phe Gln Ser Glu Ala Glu Trp Gln Lys Ala Gln Ser Gln Asn
    450                 455                 460
Ile Asn Gln Tyr Arg Pro Tyr Leu Leu Val Pro Asn Thr Asn Lys Ser
465                 470                 475                 480
Leu Gly Ile Phe Gly Leu Glu Gln Leu Lys Leu Asn Gln Met Thr Phe
                485                 490                 495
Lys Val Ala Met Arg His Glu Arg Gln Lys Thr Pro Ile Glu Tyr Asp
            500                 505                 510
Gln His Leu Leu Asp His Ala Leu Gln Tyr Phe Leu Ser Lys Ala Gln
        515                 520                 525
Leu Lys Ala Pro Asp His Pro Asp Leu Thr Thr Tyr Lys Gln His Ala
    530                 535                 540
Thr Ser Tyr Ala Gly Ser Ala Leu Trp Asp Ile Thr Pro Asn His Arg
545                 550                 555                 560
Leu Ser Leu Thr Tyr Ser His Asn Glu Arg Ile Pro Ser Pro Met Glu
                565                 570                 575
Leu Tyr Tyr Gln Gly Gly His Leu Ala Thr Ser Ser Phe Glu His Gly
            580                 585                 590
Asn Lys Asn Leu Val Lys Glu Lys Ser Asp Asn Tyr Glu Leu Gly Phe
        595                 600                 605
Met His Thr Ala Asp Lys Val Ser Tyr Lys Ala Ser Thr Tyr Tyr Ser
    610                 615                 620
Asn Phe Asp Asn Tyr Ile Phe Asn Glu Thr Ile Ala Lys Glu Gly Asn
625                 630                 635                 640
Leu Tyr Ile Arg Arg Tyr Asn Gln Thr Thr Ala Lys Phe Tyr Gly Val
                645                 650                 655
Glu Gly Ser Leu Thr Tyr Gln Pro Asn Ala Asn His Ser Val Met Phe
            660                 665                 670
Phe Gly Asp Met Val Gln Gly Lys Ile Gly Ala Leu Ser Asp Ile Lys
        675                 680                 685
Gly Lys Leu Val Tyr Ala Gly Arg Lys Trp Val Tyr Phe Asp Asp Asp
    690                 695                 700
Ile Lys Asp Met Thr Val Asp Asp Asn Gly Asp Tyr Asp Ala Asp Gly
705                 710                 715                 720
Gly Leu Thr Cys Ala Leu Lys Thr Pro Glu Gln Trp Gly Gln Ile Asn
                725                 730                 735
Asp Asn Asn Asp Cys Ser Thr Thr Ile Asn Val Tyr Lys Asn Gly Thr
            740                 745                 750
Thr Thr Ser Gly Glu Glu Asp Tyr Asp Arg Leu Ala Arg Asn Pro Thr
        755                 760                 765
Tyr Ala Pro Arg Val Pro Pro Ser Arg Leu Gly Ile Arg Trp Gln Gly
    770                 775                 780
Tyr Phe Gly Asp His Trp Ser Ala Asn Ala Glu Phe Asn His Val Phe
785                 790                 795                 800
Ala Gln Asn Lys Val Ala Thr Ser Thr Val Ala Ile Lys Pro Gln Phe
```

```
                    805                     810                     815
Lys Gln Pro Glu Gly Cys Gln Arg His Glu Ser His Cys Arg Ile Ser
                820                     825                     830
Asp Tyr Gly Ser Asp Asn Asn Pro Leu Met Met Gln Pro Arg Tyr Ile
            835                     840                     845
Thr Glu Asn Lys Thr Ala Gly Tyr Asn Leu Leu Asn Val Gly Leu Asp
    850                     855                     860
Tyr Asn Asn Ala Tyr Arg Asn Val Asp Tyr Thr Leu Ser Ile Arg Ala
865                     870                     875                     880
Asn Asn Leu Leu Asn Glu Gln Ile Tyr Ile His Asn Ser Phe Leu Pro
                    885                     890                     895
Phe Val Pro Gln Met Gly Arg Asn Leu Thr Leu Gly Leu Thr Ala Lys
                900                     905                     910
Phe
```

## Description

### FIELD OF THE INVENTION

This invention relates to polynucleotides, (herein referred to as "BASB103"), polypeptides encoded by them (referred to herein as "BASB103"), recombinant materials and methods for their production. In another aspect, the invention relates to methods for using such polypeptides and polynucleotides, including vaccines against bacterial infections. In a further aspect, the invention relates to diagnostic assays for detecting infection of certain pathogens.

### BACKGROUND OF THE INVENTION

*Moraxella catarrhalis* (also named *Branhamella catarrhalis)* is a Gram negative bacteria frequently isolated from the human upper respiratory tract. It is responsible for several pathologies the main ones being otitis media in infants and children, and pneumonia in elderlies. It is also responsible of sinusitis, nosocomial infections and less frequently of invasive diseases.

Otitis media is an important childhood disease both by the number of cases and its potential sequelae. More than 3.5 millions cases are recorded every year in the United States, and it is estimated that 80 % of the children have experienced at least one episode of otitis before reaching the age of 3 (Klein, JO (1994) Clin.Inf.Dis 19:823). Left untreated, or becoming chronic, this disease may lead to hearing losses that could be temporary (in the case of fluid accumulation in the middle ear) or permanent (if the auditive nerve is damaged). In infants, such hearing losses may be responsible for a delayed speech learning.

Three bacterial species are primarily isolated from the middle ear of children with otitis media: *Streptococcus pneumoniae,* non typeable *Haemophilus influenza* (NTHi) and *M. catarrhalis.* They are present in 60 to 90 % of the cases. A review of recent studies shows that *S. pneumoniae* and NTHi represent both about 30 %, and *M. catarrhalis* about 15 % of the otitis media cases (Murphy, TF (1996) Microbiol.Rev. 60:267). Other bacteria could be isolated from the middle ear *(H. influenza* type B, *S. pyogenes* etc) but at a much lower frequency (2 % of the cases or less).

Epidemiological data indicate that, for the pathogens found in the middle ear, the colonization of the upper respiratory tract is an absolute prerequisite for the development of an otitis; other are however also required to lead to the disease (Dickinson, DP et al. (1988) J. Infect.Dis. 158:205, Faden, HL et al. (1991) Ann.Otorhinol.Laryngol. 100: 612). These are important to trigger the migration of the bacteria into the middle ear via the Eustachian tubes, followed by the initiation of an inflammatory process. These factors are unknown todate. It has been postulated that a transient anomaly of the immune system following a viral infection, for example, could cause an inability to control the colonization of the respiratory tract (Faden, HL et al (1994) J. Infect.Dis. 169:1312). An alternative explanation is that the exposure to environmental factors allow a more important colonization of some children, who subsequently become susceptible to the development of otitis media because of the sustained presence of middle ear pathogens (Murphy, TF (1996) Microbiol.Rev. 60:267).

The immune response to *M. catarrhalis* is poorly characterized. The analysis of strains isolated sequentially from the nasopharynx of babies followed from 0 to 2 years of age, indicates that they get and eliminate frequently new strains. This indicates that an efficacious immune response against this bacteria is mounted by the colonized children (Faden, HL et al (1994) J. Infect.Dis. 169:1312).

In most adults tested, bactericidal antibodies have been identified (Chapman, AJ et al. (1985) J. Infect.Dis. 151:878). Strains of *M. catarrhalis* present variations in their capacity to resist serum bactericidal activity: in general, isolates from diseased individuals are more resistant than those who are simply colonized (Hol, C et al. (1993) Lancet 341:1381, Jordan, KL et al. (1990) Am.J.Med. 88 (suppl. 5A):28S). Serum resistance could therfore be considered as a virulence factor of the bacteria. An opsonizing activity has been observed in the sera of children recovering from otitis media.

The antigens targetted by these different immune responses in humans have not been identified, with the exception of OMP B1, a 84 kDa protein which expression is regulated by iron, and that is recognized by the sera of patients with pneumonia (Sethi, S, et al. (1995) Infect.Immun. 63:1516), and of UspA1 and UspA2 (Chen D. et al.(1999), Infect.Immun. 67:1310).

A few other membrane proteins present on the surface of M. *catarrhalis* have been characterized using biochemical method, or for their potential implication in the induction of a protective immunity (for review, see Murphy, TF (1996) Microbiol.Rev. 60:267). In a mouse pneumonia model, the presence of antibodies raised against some of them (UspA, CopB) favors a faster clearance of the pulmonary infection. Another polypeptide (OMP CD) is highly conserved among M *catarrhalis* strains, and presents homologies with a porin of *Pseudomonas aeruginosa,* which has been demonstrated efficacious against this bacterium in animal models.

The frequency of *Moraxella catarrhalis* infections has risen dramatically in the past few decades. This has been attributed to the emergence of multiply antibiotic resistant strains and an increasing population of people with weakened immune systems. It is no longer uncommon to isolate *Moroxella catarrhalis* strains that are resistant to some or all of the standard antibiotics. This phenomenon has created an unmet medical need and demand for new anti-microbial agents, vaccines, drug screening methods, and diagnostic tests for this organism.

## SUMMARY OF THE INVENTION

The present invention relates to BASB103 in particular BASB103 polypeptides and BASB 103 polynucleotides, recombinant materials and methods for their production. In another aspect, the invention relates to methods for using such polypeptides and polynucleotides, including prevention and treatment of microbial diseases, amongst others. In a further aspect, the invention relates to diagnostic assays for detecting diseases associated with microbial infections and conditions associated with such infections, such as assays for detecting expression or activity of BASB103 polynucleotides or polypeptides.

Various changes and modifications within the spirit and scope of the disclosed invention will become readily apparent to those skilled in the art from reading the following descriptions and from reading the other parts of the present disclosure.

## DESCRIPTION OF THE INVENTION

The invention relates to BASB 103 polypeptides and polynucleotides as described in greater detail below. The polypeptides according to the invention all belong to the family of Outer Membrane Proteins and are therefore useful as vaccine candidates.

In particular, the invention relates to polypeptides and polynucleotides of BASB 103 of *Moraxella catarrhalis,* which is related by amino acid sequence homology to no known protein but has some features of outer membrane protein: signal sequence, aromatic amino acid N-terminal, high beta-strand 2D structure prediction. The invention relates especially to BASB103 having the nucleotide and amino acid sequences set out in SEQ ID NO:1 and SEQ ID NO:2 respectively.

It is understood that sequences recited in the Sequence Listing below as "DNA" represent an exemplification of one embodiment of the invention, since those of ordinary skill will recognize that such sequences can be usefully employed in polynucleotides in general. including ribopolynucleotides.

### <u>Polypeptides</u>

In one aspect of the invention there are provided polypeptides of *Moraxella catarrhalis* referred to herein as "BASB103" and "BASB103" polypeptides" as well as biologically, diagnostically, prophylactically, clinically or therapeutically useful variants thereof, and compositions comprising the same.

The present invention further provides for:

(a) an isolated polypeptide which comprises an amino acid sequence which has at least 85% identity, preferably at least 90% identity, more preferably at least 95% identity, most preferably at least 97-99% or exact identity, to that of SEQ ID NO:2;

(b) a polypeptide encoded by an isolated polynucleotide comprising a polynucleotide sequence which has at least 85% identity, preferably at least 90% identity, more preferably at least 95% identity, even more preferably at least 97-99% or exact identity to SEQ ID NO:1 over the entire length of SEQ ID NO:1; or

(c) a polypeptide encoded by an isolated polynucleotide comprising a polynucleotide sequence encoding a polypeptide which has at least 85% identity, preferably at least 90% identity, more preferably at least 95% identity, even more preferably at least 97-99% or exact identity, to the amino acid sequence of SEQ ID NO:2.

The BASB103 polypeptides provided in SEQ ID NO:2 are the BASB103 polypeptides from *Moraxella catarrhalis* strain Mc2931 (ATCC 43617).

The invention also provides an immunogenic fragment of a BASB103 polypeptide, that is, a contiguous portion of the BASB103, polypeptide which has the same or substantially the same immunogenic activity as the polypeptide comprising the amino acid sequence of SEQ ID NO:2 with the proviso that said fragment does not have the sequence nvvtntgatv vdgtrtifst lvkpaawaa v. That is to say, the fragment (if necessary when coupled to a carrier) is capable of raising an immune response which recognises the BASB103 polypeptide. Such an immunogenic fragment may include, for example, the BASB103 polypeptide lacking an N-terminal leader sequence, and/or a transmembrane domain and/or a C-terminal anchor domain. In a preferred aspect the immunogenic fragment of BASB103 according to the invention comprises substantially all of the extracellular domain of a polypeptide which has at least 85% identity, preferably at least 90% identity, more preferably at least 95% identity, most preferably at least 97-99% identity, to that of SEQ ID NO:

2 over the entire length of SEQ ID NO:2.

A fragment is a polypeptide having an amino acid sequence that is entirely the same as part but not all of any amino acid sequence of any polypeptide of the invention. As with BASB 103 polypeptides, fragments may be "free-standing," or comprised within a larger polypeptide of which they form a part or region, most preferably as a single continuous region in a single larger polypeptide.

Preferred fragments include, for example, truncation polypeptides having a portion of an amino acid sequence of SEQ ID NO:2 or of variants thereof, such as a continuous series of residues that includes an amino- and/or carboxyl-terminal amino acid sequence. Degradation forms of the polypeptides of the invention produced by or in a host cell, are also preferred. Further preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions.

Further preferred fragments include an isolated polypeptide comprising an amino acid sequence having at least 15, 20, 30, 40, 50 or 100 contiguous amino acids from the amino acid sequence of SEQ ID NO:2, or an isolated polypeptide comprising an amino acid sequence having at least 15, 20, 30, 40, 50 or 100 contiguous amino acids truncated or deleted from the amino acid sequence of SEQ ID NO:2.

Fragments of the polypeptides of the invention may be employed for producing the corresponding full-length polypeptide by peptide synthesis; therefore, these fragments may be employed as intermediates for producing the full-length polypeptides of the invention.

Particularly preferred are variants in which several, 5-10, 1-5, 1-3, 1-2 or 1 amino acids are substituted, deleted, or added in any combination.

The polypeptides, or immunogenic fragments, of the invention may be in the form of the "mature" protein or may be a part of a larger protein such as a precursor or a fusion protein. It is often advantageous to include an additional amino acid sequence which contains secretory or leader sequences, pro-sequences, sequences which aid in purification such as multiple histidine residues, or an additional sequence for stability during recombinant production. Furthermore, addition of exogenous polypeptide or lipid tail or polynucleotide sequences to increase the immunogenic potential of the final molecule is also considered.

In one aspect, the invention relates to genetically engineered soluble fusion proteins comprising a polypeptide of the present invention, or a fragment thereof, with the proviso that said fusion protein does not have the sequence nvvtntgatv vdgtrtifst lvkpaawaa v and various portions of the constant regions of heavy or light chains of immunoglobulins of various subclasses (IgG, IgM, IgA, IgE). Preferred as an immunoglobulin is the constant part of the heavy chain of human IgG, particularly IgGI, where fusion takes place at the hinge region. In a particular embodiment, the Fc part can be removed simply by incorporation of a cleavage sequence which can be cleaved with blood clotting factor Xa.

Furthermore, this invention relates to processes for the preparation of these fusion proteins by genetic engineering, and to the use thereof for drug screening, diagnosis and therapy. A further aspect of the invention also relates to polynucleotides encoding such fusion proteins. Examples of fusion protein technology can be found in International Patent Application Nos. WO94/29458 and WO94/22914.

The proteins may be chemically conjugated, or expressed as recombinant fusion proteins allowing increased levels to be produced in an expression system as compared to non-fused protein. The fusion partner may assist in providing T helper epitopes (immunological fusion partner), preferably T helper epitopes recognised by humans, or assist in expressing the protein (expression enhancer) at higher yields than the native recombinant protein. Preferably the fusion partner will be both an immunological fusion partner and expression enhancing partner.

Fusion partners include protein D from *Haemophilus influenzae* and the non-structural protein from influenzae virus, NS 1 (hemagglutinin). Another fusion partner is the protein known as LytA. Preferably the C terminal portion of the molecule is used. Lyta is derived from *Streptococcus pneumoniae* which synthesize an N-acetyl-L-alanine amidase, amidase LytA, (coded by the lytA gene {Gene, 43 (1986) page 265-272}) an autolysin that specifically degrades certain bonds in the peptidoglycan backbone. The C-terminal domain of the LytA protein is responsible for the affinity to the choline or to some choline analogues such as DEAE. This property has been exploited for the development of E.coli C-LytA expressing plasmids useful for expression of fusion proteins. Purification of hybrid proteins containing the C-LytA fragment at its amino terminus has been described (Biotechnology: 10, (1992) page 795-798). It is possible to use the repeat portion of the LytA molecule found in the C terminal end starting at residue 178, for example residues 188 - 305.

The present invention also includes variants of the aforementioned polypeptides, that is polypeptides that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr.

Polypeptides of the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

It is most preferred that a polypeptide of the invention is derived from *Moraxella catarrhalis,* however, it may preferably be obtained from other organisms of the same taxonomic genus. A polypeptide of the invention may also be obtained, for example, from organisms of the same taxonomic family or order.

## Polynucleotides

It is an object of the invention to provide polynucleotides that encode BASB103 polypeptides, particularly polynucleotides that encode the polypeptide herein designated BASB 103.

In a particularly preferred embodiment of the invention the polynucleotide comprises a region encoding BASB103 polypeptides comprising a sequence set out in SEQ ID NO:1 which includes a full length gene, or a variant thereof.

The BASB103 polynucleotides provided in SEQ ID NO:1 the BASB 103, polynucleotides from *Moraxella catarrhalis* strain Mc2931 (ATCC 43617).

As a further aspect of the invention there are provided isolated nucleic acid molecules encoding and/or expressing BASB 103 polypeptides and polynucleotides, particularly *Moraxella catarrhalis* BASB 103 polypeptides and polynucleotides, including, for example, unprocessed RNAs, ribozyme RNAs, mRNAs, cDNAs, genomic DNAs, B- and Z-DNAs. Further embodiments of the invention include biologically, diagnostically, prophylactically, clinically or therapeutically useful polynucleotides and polypeptides, and variants thereof, and compositions comprising the same.

Another aspect of the invention relates to isolated polynucleotides, including at least one full length gene, that encodes a BASB103 polypeptide having a deduced amino acid sequence of SEQ ID NO:2 and polynucleotides closely related thereto and variants thereof.

In another particularly preferred embodiment of the invention there is a BASB 103, polypeptide from *Moraxella catarrhalis* comprising or consisting of an amino acid sequence of SEQ ID NO:2 or a variant thereof.

Using the information provided herein, such as a polynucleotide sequence set out in SEQ ID NO: a polynucleotide of the invention encoding BASB 103 polypeptide may be obtained using standard cloning and screening methods, such as those for cloning and sequencing chromosomal DNA fragments from bacteria using *Moraxella catarrhalis* Catlin cells as starting material, followed by obtaining a full length clone. For example, to obtain a polynucleotide sequence of the invention, such as a polynucleotide sequence given in SEQ ID NO:1, typically a library of clones of chromosomal DNA of *Moraxella catarrhalis* Catlin in *E.coli* or some other suitable host is probed with a radiolabeled oligonucleotide, preferably a 17-mer or longer, derived from a partial sequence. Clones carrying DNA identical to that of the probe can then be distinguished using stringent hybridization conditions. By sequencing the individual clones thus identified by hybridization with sequencing primers designed from the original polypeptide or polynucleotide sequence it is then possible to extend the polynucleotide sequence in both directions to determine a full length gene sequence. Conveniently, such sequencing is performed, for example, using denatured double stranded DNA prepared from a plasmid clone. Suitable techniques are described by Maniatis, T., Fritsch, E.F. and Sambrook et al., MOLECULAR CLONING, A LABORATORYMANUAL, 2nd Ed.; Cold Spring Harbor Laboratory Press, Cold Spring Harbor, New York (1989). (see in particular Screening By Hybridization 1.90 and Sequencing Denatured Double-Stranded DNA Templates 13.70). Direct genomic DNA sequencing may also be performed to obtain a full length gene sequence. Illustrative of the invention, each polynucleotide set out in SEQ ID NO:1 was discovered in a DNA library derived from *Moraxella catarrhalis.*

Moreover, each DNA sequence set out in SEQ ID NO:1 contains an open reading frame encoding a protein having about the number of amino acid residues set forth in SEQ ID NO:2 with a deduced molecular weight that can be calculated using amino acid residue molecular weight values well known to those skilled in the art.

The polynucleotide of SEQ ID NO: 1, between the start codon at nucleotide number 1 and the stop codon which begins at nucleotide number 757 of SEQ ID NO:1, encodes the polypeptide of SEQ ID NO:2.

In a further aspect, the present invention provides for an isolated polynucleotide comprising or consisting of:

(a) a polynucleotide sequence which has at least 85% identity, preferably at least 90% identity, more preferably at least 95% identity, even more preferably at least 97-99% or exact identity to SEQ ID NO:1 over the entire length of SEQ ID NO:1, respectively; or

(b) a polynucleotide sequence encoding a polypeptide which has at least 85% identity, preferably at least 90% identity, more preferably at least 95% identity, even more preferably at least 97-99% or 100% exact, to the amino acid sequence of SEQ ID NO:2, over the entire length of SEQ ID NO:2 respectively.

A polynucleotide encoding a polypeptide of the present invention, including homologs and orthologs from species other than *Moraxella catarrhalis,* may be obtained by a process which comprises the steps of screening an appropriate library under stringent hybridization conditions (for example, using a temperature in the range of 45 - 65°C and an SDS concentration from 0.1 - 1%) with a labeled or detectable probe consisting of or comprising the sequence of SEQ ID NO:1 or a fragment thereof; and isolating a full-length gene and/or genomic clones containing said polynucleotide sequence.

The invention provides a polynucleotide sequence identical over its entire length to a coding sequence (open reading frame) in SEQ ID NO:1. Also provided by the invention is a coding sequence for a mature polypeptide or a fragment thereof, by itself as well as a coding sequence for a mature polypeptide or a fragment in reading frame with another coding sequence, such as a sequence encoding a leader or secretory sequence, a pre-, or pro- or prepro-protein sequence. The polynucleotide of the invention may also contain at least one non-coding sequence, including for example, but not limited to at least one non-coding 5' and 3' sequence, such as the transcribed but non-translated sequences, termination signals (such as rho-dependent and rho-independent termination signals), ribosome binding sites, Kozak sequences, sequences that stabilize mRNA, introns, and polyadenylation signals. The polynucleotide sequence may also comprise additional coding sequence encoding additional amino acids. For example, a marker sequence that facilitates purification of the fused polypeptide can be encoded. In certain embodiments of the invention, the marker sequence is a hexa-histidine peptide, as provided in the pQE vector (Qiagen, Inc.) and described in Gentz et al.. Proc. Natl. Acad. Sci., USA 86: 821-824 (1989), or an HA peptide tag (Wilson et al., Cell 37: 767 (1984), both of which may be useful in purifying polypeptide sequence fused to them. Polynucleotides of the invention also include, but are not limited to, polynucleotides comprising a structural gene and its naturally associated sequences that control gene expression.

The nucleotide sequence encoding BASB103, polypeptide of SEQ ID NO:2 may be identical to the polypeptide encoding sequence contained in nucleotides 1 to 756 of SEQ ID NO:1. Alternatively the nucleotide sequence encoding BASB 103, of SEQ ID NO:2, may be a sequence, which as a result of the redundancy (degeneracy) of the genetic code, also encodes the polypeptide of SEQ ID NO:2, respectively.

The term "polynucleotide encoding a polypeptide" as used herein encompasses polynucleotides that include a sequence encoding a polypeptide of the invention, particularly a bacterial polypeptide and more particularly a polypeptide of the *Moraxella catarrhalis* BASB103 having an amino acid sequence set out in SEQ ID NO:2. The term also encompasses polynucleotides that include a single continuous region or discontinuous regions encoding the polypeptide (for example, polynucleotides interrupted by integrated phage, an integrated insertion sequence, an integrated vector sequence, an integrated transposon sequence, or due to RNA editing or genomic DNA reorganization) together with additional regions, that also may contain coding and/or non-coding sequences.

The invention further relates to variants of the polynucleotides described herein that encode variants of a polypeptide having a deduced amino acid sequence of SEQ ID NO:2. Fragments of polynucleotides of the invention may be used, for example, to synthesize full-length polynucleotides of the invention.

Further particularly preferred embodiments are polynucleotides encoding BASB 103, variants, that have the amino acid sequence of BASB103 polypeptide of SEQ ID NO:2 respectively in which several, a few, 5 to 10, 1 to 5, 1 to 3, 2, 1 or no amino acid residues are substituted, modified, deleted and/or added, in any combination. Especially preferred among these are silent substitutions, additions and deletions, that do not alter the properties and activities of BASB103 polypeptide.

Further preferred embodiments of the invention are polynucleotides that are at least 85% identical over their entire length to a polynucleotide encoding BASB103 polypeptide having an amino acid sequence set out in SEQ ID NO: 2, and polynucleotides that are complementary to such polynucleotides. Alternatively, most highly preferred are polynucleotides that comprise a region that is at least 90% identical over its entire length to a polynucleotide encoding BASB 103 polypeptide and polynucleotides complementary thereto. In this regard, polynucleotides at least 95% identical over their entire length to the same are particularly preferred. Furthermore, those with at least 97% are highly preferred among those with at least 95%, and among these those with at least 98% and at least 99% are particularly highly preferred, with at least 99% being the more preferred.

Preferred embodiments are polynucleotides encoding polypeptides that retain substantially the same biological function or activity as the mature polypeptide encoded by a DNA of SEQ ID NO:1.

In accordance with certain preferred embodiments of this invention there are provided polynucleotides that hybridize, particularly under stringent conditions, to BASB 103. polynucleotide sequences, such as those polynucleotides in SEQ ID NO:1.

The invention further relates to polynucleotides that hybridize to the polynucleotide sequences provided herein. In this regard, the invention especially relates to polynucleotides that hybridize under stringent conditions to the polynucleotides described herein. As herein used, the terms "stringent conditions" and "stringent hybridization conditions" mean hybridization occurring only if there is at least 95% and preferably at least 97% identity between the sequences. A specific example of stringent hybridization conditions is overnight incubation at 42°C in a solution comprising: 50% formamide, 5x SSC (150mM NaCl, 15mM trisodium citrate), 50 mM sodium phosphate (pH7.6), 5x Denhardt's solution, 10% dextran sulfate, and 20 micrograms/ml of denatured, sheared salmon sperm DNA, followed by washing the hybridization support in 0.1x SSC at about 65°C. Hybridization and wash conditions are well known and exemplified in Sambrook, et al., Molecular Cloning: A Laboratory Manual, Second Edition, Cold Spring Harbor, N.Y., (1989), particularly Chapter 11 therein. Solution hybridization may also be used with the polynucleotide sequences provided by the invention.

The invention also provides a polynucleotide consisting of or comprising a polynucleotide sequence obtained by screening an appropriate library containing the complete gene for a polynucleotide sequence set forth in SEQ ID NO: 1 under stringent hybridization conditions with a probe having the sequence of said polynucleotide sequence set forth in SEQ ID NO: 1 or a fragment thereof; and isolating said polynucleotide sequence. Fragments useful for obtaining such a polynucleotide include, for example, probes and primers fully described elsewhere herein.

As discussed elsewhere herein regarding polynucleotide assays of the invention, for instance, the polynucleotides of the invention, may be used as a hybridization probe for RNA, cDNA and genomic DNA to isolate full-length cDNAs and genomic clones encoding BASB103 and to isolate cDNA and genomic clones of other genes that have a high identity, particularly high sequence identity, to the BASB103 gene. Such probes generally will comprise at least 15 nucleotide residues or base pairs. Preferably, such probes will have at least 30 nucleotide residues or base pairs and may have at least 50 nucleotide residues or base pairs. Particularly preferred probes will have at least 20 nucleotide residues or base pairs and will have less than 30 nucleotide residues or base pairs.

A coding region of a BASB103 gene may be isolated by screening using a DNA sequence provided in SEQ ID NO:1 to synthesize an oligonucleotide probe. A labeled oligonucleotide having a sequence complementary to that of a gene of the invention is then used to screen a library of cDNA, genomic DNA or mRNA to determine which members of the library the probe hybridizes to.

There are several methods available and well known to those skilled in the art to obtain full-length DNAs, or extend short DNAs, for example those based on the method of Rapid Amplification of cDNA ends (RACE) (see, for example, Frohman, et al., PNAS USA 85: 8998-9002, 1988). Recent modifications of the technique, exemplified by the Marathon™ technology (Clontech Laboratories Inc.) for example, have significantly simplified the search for longer cDNAs. In the Marathon™ technology, cDNAs have been prepared from mRNA extracted from a chosen tissue and an 'adaptor' sequence ligated onto each end. Nucleic acid amplification (PCR) is then carried out to amplify the "missing" 5' end of the DNA using a combination of gene specific and adaptor specific oligonucleotide primers. The PCR reaction is then repeated using "nested" primers, that is, primers designed to anneal within the amplified product (typically an adaptor specific primer that anneals further 3' in the adaptor sequence and a gene specific primer that anneals further 5' in the selected gene sequence). The products of this reaction can then be analyzed by DNA sequencing and a full-length DNA constructed either by joining the product directly to the existing DNA to give a complete sequence, or carrying out a separate full-length PCR using the new sequence information for the design of the 5' primer.

The polynucleotides and polypeptides of the invention may be employed, for example, as research reagents and materials for discovery of treatments of and diagnostics for diseases, particularly human diseases, as further discussed herein relating to polynucleotide assays.

The polynucleotides of the invention that are oligonucleotides derived from a sequence of SEQ ID NOS:1 may be used in the processes herein as described, but preferably for PCR, to determine whether or not the polynucleotides identified herein in whole or in part are transcribed in bacteria in infected tissue. It is recognized that such sequences will also have utility in diagnosis of the stage of infection and type of infection the pathogen has attained.

The invention also provides polynucleotides that encode a polypeptide that is the mature protein plus additional amino or carboxyl-terminal amino acids, or amino acids interior to the mature polypeptide (when the mature form has more than one polypeptide chain, for instance). Such sequences may play a role in processing of a protein from precursor to a mature form, may allow protein transport, may lengthen or shorten protein half-life or may facilitate manipulation of a protein for assay or production, among other things. As generally is the case *in vivo,* the additional amino acids may be processed away from the mature protein by cellular enzymes.

For each and every polynucleotide of the invention there is provided a polynucleotide complementary to it. It is preferred that these complementary polynucleotides are fully complementary to each polynucleotide with which they are complementary.

A precursor protein, having a mature form of the polypeptide fused to one or more prosequences may be an inactive form of the polypeptide. When prosequences are removed such inactive precursors generally are activated. Some or all of the prosequences may be removed before activation. Generally, such precursors are called proproteins.

In addition to the standard A, G, C, T/U representations for nucleotides, the term "N" may also be used in describing certain polynucleotides of the invention. "N" means that any of the four DNA or RNA nucleotides may appear at such a designated position in the DNA or RNA sequence, except it is preferred that N is not a nucleic acid that when taken in combination with adjacent nucleotide positions, when read in the correct reading frame, would have the effect of generating a premature termination codon in such reading frame.

In sum, a polynucleotide of the invention may encode a mature protein, a mature protein plus a leader sequence (which may be referred to as a preprotein), a precursor of a mature protein having one or more prosequences that are not the leader sequences of a preprotein, or a preproprotein, which is a precursor to a proprotein, having a leader sequence and one or more prosequences, which generally are removed during processing steps that produce active and mature forms of the polypeptide.

In accordance with an aspect of the invention, there is provided the use of a polynucleotide of the invention for therapeutic or prophylactic purposes, in particular genetic immunization.

The use of a polynucleotide of the invention in genetic immunization will preferably employ a suitable delivery method such as direct injection of plasmid DNA into muscles (Wolff et al.. Hum Mol Genet (1992) 1: 363, Manthorpe et al., Hum. Gene Ther. (1983) 4: 419), delivery of DNA complexed with specific protein carriers *(*Wu et al., J Biol Chem. (1989) 264: 16985), coprecipitation of DNA with calcium phosphate (Benvenisty & Reshef, PNAS USA, (1986) 83: 9551), encapsulation of DNA in various forms of liposomes (Kaneda et al., Science (1989) 243: 375), particle bombardment *(*Tang et al. Nature (1992) 356:152, Eisenbraun et al., DNA Cell Biol (1993) 12: 791) and *in vivo* infection using cloned retroviral vectors (Seeger et al., PNAS USA (1984) 81: 5849).

## Vectors, Host Cells, Expression Systems

The invention also relates to vectors that comprise a polynucleotide or polynucleotides of the invention, host cells that are genetically engineered with vectors of the invention and the production of polypeptides of the invention by recombinant techniques. Cell-free translation systems can also be employed to produce such proteins using RNAs derived from the DNA constructs of the invention.

Recombinant polypeptides of the present invention may be prepared by processes well known in those skilled in the art from genetically engineered host cells comprising expression systems. Accordingly, in a further aspect, the present invention relates to expression systems that comprise a polynucleotide or polynucleotides of the present invention, to host cells which are genetically engineered with such expression systems, and to the production of polypeptides of the invention by recombinant techniques.

For recombinant production of the polypeptides of the invention, host cells can be genetically engineered to incorporate expression systems or portions thereof or polynucleotides of the invention. Introduction of a polynucleotide into the host cell can be effected by methods described in many standard laboratory manuals, such as Davis, et al., BASIC METHODS IN MOLECULAR BIOLOGY, (1986) and Sambrook, et al., MOLECULAR CLONING: A LABORATORY MANUAL, 2nd Ed., Cold Spring Harbor Laboratory Press, Cold Spring Harbor, N.Y. (1989), such as, calcium phosphate transfection, DEAE-dextran mediated transfection, transvection, microinjection, cationic lipid-mediated transfection, electroporation, transduction, scrape loading, ballistic introduction and infection.

Representative examples of appropriate hosts include bacterial cells, such as cells of streptococci, staphylococci, enterococci, *E. coli,* streptomyces, cyanobacteria, *Bacillus subtilis, Neisseria meningitidis* and *Moraxella catarrhalis;* fungal cells, such as cells of a yeast, *Kluveromyces, Saccharomyces,* a basidiomycete, *Candida albicans* and *Aspergillus;* insect cells such as cells of *Drosophila* S2 and *Spodoptera* Sf9; animal cells such as CHO, COS, HeLa, C127, 3T3, BHK, 293, CV-1 and Bowes melanoma cells; and plant cells, such as cells of a gymnosperm or angiosperm.

A great variety of expression systems can be used to produce the polypeptides of the invention. Such vectors include, among others, chromosomal-, episomal- and virus-derived vectors, for example, vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses, picornaviruses, retroviruses, and alphaviruses and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook et al., MOLECULAR CLONING, A LABORATORY MANUAL*, (supra).*

In recombinant expression systems in eukaryotes, for secretion of a translated protein into the lumen of the endoplasmic reticulum, into the periplasmic space or into the extracellular environment, appropriate secretion signals may be incorporated into the expressed polypeptide. These signals may be endogenous to the polypeptide or they may be heterologous signals.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, ion metal affinity chromatography (IMAC) is employed for purification. Well known techniques for refolding proteins may be employed to regenerate active conformation when the polypeptide is denatured during intracellular synthesis, isolation and or purification.

The expression system may also be a recombinant live microorganism, such as a virus or bacterium. The gene of interest can be inserted into the genome of a live recombinant virus or bacterium. Inoculation and *in vivo* infection with this live vector will lead to *in vivo* expression of the antigen and induction of immune responses. Viruses and bacteria used for this purpose are for instance: poxviruses (e.g; vaccinia, fowlpox, canarypox), alphaviruses (Sindbis virus, Semliki Forest Virus, Venezuelian Equine Encephalitis Virus), adenoviruses, adeno-associated virus, picornaviruses (poliovirus, rhinovirus), herpesviruses (varicella zoster virus, etc), Listeria, Salmonella, Shigella, BCG. These viruses and bacteria can be virulent, or attenuated in various ways in order to obtain live vaccines. Such live vaccines also form part of the invention.

## Diagnostic, Prognostic, Serotyping and Mutation Assays

This invention is also related to the use of BASB103 polynucleotides and polypeptides of the invention for use as diagnostic reagents. Detection of BASB103 polynucleotides and/or polypeptides in a eukaryote, particularly a mammal, and especially a human, will provide a diagnostic method for diagnosis of disease, staging of disease or response of an infectious organism to drugs. Eukaryotes, particularly mammals, and especially humans, particularly those infected or suspected to be infected with an organism comprising the BASB103 gene or protein, may be detected at the nucleic acid or amino acid level by a variety of well known techniques as well as by methods provided herein.

Polypeptides and polynucleotides for prognosis, diagnosis or other analysis may be obtained from a putatively infected and/or infected individual's bodily materials. Polynucleotides from any of these sources, particularly DNA or RNA, may be used directly for detection or may be amplified enzymatically by using PCR or any other amplification technique prior to analysis. RNA, particularly mRNA, cDNA and genomic DNA may also be used in the same ways. Using amplification, characterization of the species and strain of infectious or resident organism present in an individual, may be made by an analysis of the genotype of a selected polynucleotide of the organism. Deletions and insertions can be detected by a change in size of the amplified product in comparison to a genotype of a reference sequence selected from a related organism, preferably a different species of the same genus or a different strain of the same species. Point mutations can be identified by hybridizing amplified DNA to labeled BASB103 polynucleotide sequences. Perfectly or significantly matched sequences can be distinguished from imperfectly or more significantly mismatched duplexes by DNase or RNase digestion, for DNA or RNA respectively, or by detecting differences in melting temperatures or renaturation kinetics. Polynucleotide sequence differences may also be detected by alterations in the electrophoretic mobility of polynucleotide fragments in gels as compared to a reference sequence. This may be carried out with or without denaturing agents. Polynucleotide differences may also be detected by direct DNA or RNA sequencing. See, for example, Myers et al., Science, 230: 1242 (1985). Sequence changes at specific locations also may be revealed by nuclease protection assays, such as RNase, V I and S 1 protection assay or a chemical cleavage method. See, for example. Cotton et al., Proc. Natl. Acad. Sci., USA, 85: 4397-4401 (1985).

In another embodiment, an array of oligonucleotides probes comprising BASB 103 nucleotide sequence or fragments thereof can be constructed to conduct efficient screening of, for example, genetic mutations, serotype, taxonomic classification or identification. Array technology methods are well known and have general applicability and can be used to address a variety of questions in molecular genetics including gene expression, genetic linkage, and genetic variability (see, for example, Chee et al., Science, 274: 610 (1996)).

Thus in another aspect, the present invention relates to a diagnostic kit which comprises:

(a) a polynucleotide of the present invention, preferably the nucleotide sequence of SEQ ID NO:1, or a fragment thereof ;
(b) a nucleotide sequence complementary to that of (a);
(c) a polypeptide of the present invention, preferably the polypeptide of SEQ ID NO:2 or a fragment thereof; or
(d) an antibody to a polypeptide of the present invention, preferably to the polypeptide of SEQ ID NO:2.

It will be appreciated that in any such kit, (a), (b), (c) or (d) may comprise a substantial component. Such a kit will be of use in diagnosing a disease or susceptibility to a Disease, among others.

This invention also relates to the use of polynucleotides of the present invention as diagnostic reagents. Detection of a mutated form of a polynucleotide of the invention, preferably SEQ ID NO:1, which is associated with a disease or pathogenicity will provide a diagnostic tool that can add to, or define, a diagnosis of a disease, a prognosis of a course of disease, a determination of a stage of disease, or a susceptibility to a disease, which results from under-expression, over-expression or altered expression of the polynucleotide. Organisms, particularly infectious organisms, carrying mutations in such polynucleotide may be detected at the polynucleotide level by a variety of techniques, such as those described elsewhere herein.

Cells from an organism carrying mutations or polymorphisms (allelic variations) in a polynucleotide and/or polypeptide of the invention may also be detected at the polynucleotide or polypeptide level by a variety of techniques, to allow for serotyping, for example. For example, RT-PCR can be used to detect mutations in the RNA. It is particularly preferred to use RT-PCR in conjunction with automated detection systems, such as, for example, GeneScan. RNA, cDNA or genomic DNA may also be used for the same purpose, PCR. As an example, PCR primers complementary to a polynucleotide encoding BASB103 polypeptide can be used to identify and analyze mutations.

The invention further provides primers with 1, 2, 3 or 4 nucleotides removed from the 5' and/or the 3' end. These primers may be used for, among other things, amplifying BASB103 DNA and/or RNA isolated from a sample derived from an individual, such as a bodily material. The primers may be used to amplify a polynucleotide isolated from an infected individual, such that the polynucleotide may then be subject to various techniques for elucidation of the polynucleotide sequence. In this way, mutations in the polynucleotide sequence may be detected and used to diagnose and/or prognose the infection or its stage or course, or to serotype and/or classify the infectious agent.

The invention further provides a process for diagnosing, disease, preferably bacterial infections, more preferably infections caused by *Moraxella catarrhalis,* comprising determining from a sample derived from an individual, such as a bodily material, an increased level of expression of polynucleotide having a sequence of SEQ ID NO:1. Increased or decreased expression of a BASB103 polynucleotide can be measured using any on of the methods well known in the art for the quantitation of polynucleotides, such as, for example, amplification, PCR, RT-PCR, RNase protection, Northern blotting, spectrometry and other hybridization methods.

In addition, a diagnostic assay in accordance with the invention for detecting over-expression of BASB 103 polypeptide compared to normal control tissue samples may be used to detect the presence of an infection, for example. Assay techniques that can be used to determine levels of a BASB103 polypeptide, in a sample derived from a host, such as a bodily material, are well-known to those of skill in the art. Such assay methods include radioimmunoassays, competitive-binding assays, Western Blot analysis, antibody sandwich assays, antibody detection and ELISA assays.

The polynucleotides of the invention may be used as components of polynucleotide arrays, preferably high density arrays or grids. These high density arrays are particularly useful for diagnostic and prognostic purposes. For example, a set of spots each comprising a different gene, and further comprising a polynucleotide or polynucleotides of the invention, may be used for probing, such as using hybridization or nucleic acid amplification, using a probes obtained or derived from a bodily sample, to determine the presence of a particular polynucleotide sequence or related sequence in an individual. Such a presence may indicate the presence of a pathogen, particularly *Moraxella catarrhalis,* and may be useful in diagnosing and/or prognosing disease or a course of disease. A grid comprising a number of variants of the polynucleotide sequence of SEQ ID NO: 1 are preferred. Also preferred is a comprising a number of variants of a polynucleotide sequence encoding the polypeptide sequence of SEQ ID NO:2.

## Antibodies

The polypeptides and polynucleotides of the invention or variants thereof, or cells expressing the same can be used as immunogens to produce antibodies immunospecific for such polypeptides or polynucleotides respectively.

In certain preferred embodiments of the invention there are provided antibodies against BASB103 polypeptides or polynucleotides.

Antibodies generated against the polypeptides or polynucleotides of the invention can be obtained by administering the polypeptides and/or polynucleotides of the invention, or epitope-bearing fragments of either or both, analogues of either or both, or cells expressing either or both, to an animal, preferably a nonhuman, using routine protocols. For preparation of monoclonal antibodies, any technique known in the art that provides antibodies produced by continuous cell line cultures can be used. Examples include various techniques, such as those in Kohler, G. and Milstein, C., Nature 256: 495-497 (1975); Kozbor et al., Immunology Today 4: 72 (1983); Cole et al., pg. 77-96 in MONOCLONAL ANTIBODIES AND CANCER THERAPY, Alan R. Liss; Inc. (1985).

Techniques for the production of single chain antibodies (U.S. Patent No. 4,946,778) can be adapted to produce single chain antibodies to polypeptides or polynucleotides of this invention. Also, transgenic mice, or other organisms or animals, such as other mammals, may be used to express humanized antibodies immunospecific to the polypeptides or polynucleotides of the invention.

Alternatively, phage display technology may be utilized to select antibody genes with binding activities towards a polypeptide of the invention either from repertoires of PCR amplified v-genes of lymphocytes from humans screened for possessing anti-BASB 103. or from naive libraries (McCafferty, et al., (1990), Nature 348, 552-554; Marks, et al., (1992) Biotechnologv 10. 779-783). The affinity of these antibodies can also be improved by, for example, chain shuffling (Clackson et al., (1991) Nature 352: 628).

The above-described antibodies may be employed to isolate or to identify clones expressing the polypeptides or polynucleotides of the invention to purify the polypeptides or polynucleotides by, for example, affinity chromatography.

Thus, among others, antibodies against BASB103 polypeptide or BASB103 polynucleotide may be employed to treat infections, particularly bacterial infections.

Polypeptide variants include antigenically, epitopically or immunologically equivalent variants form a particular aspect of this invention.

Preferably, the antibody or variant thereof is modified to make it less immunogenic in the individual. For example, if the individual is human the antibody may most preferably be "humanized," where the complimentarity determining region or regions of the hybridomaderived antibody has been transplanted into a human monoclonal antibody, for example as described in Jones et al. (1986), Nature 321, 522-525 or Tempest et al., (1991) Biotechnology 9, 266-273.

**Antagonists and Agonists - Assays and Molecules**

Polypeptides and polynucleotides of the invention may also be used to assess the binding of small molecule substrates and ligands in, for example, cells, cell-free preparations, chemical libraries, and natural product mixtures. These substrates and ligands may be natural substrates and ligands or may be structural or functional mimetics. See, e.g., Coligan et al., Current Protocols in Immunology 1(2): Chapter 5 (1991).

The screening methods may simply measure the binding of a candidate compound to the polypeptide or polynucleotide, or to cells or membranes bearing the polypeptide or polynucleotide, or a fusion protein of the polypeptide by means of a label directly or indirectly associated with the candidate compound. Alternatively, the screening method may involve competition with a labeled competitor. Further, these screening methods may test whether the candidate compound results in a signal generated by activation or inhibition of the polypeptide or polynucleotide, using detection systems appropriate to the cells comprising the polypeptide or polynucleotide. Inhibitors of activation are generally assayed in the presence of a known agonist and the effect on activation by the agonist by the presence of the candidate compound is observed. Constitutively active polypeptide and/or constitutively expressed polypeptides and polynucleotides may be employed in screening methods for inverse agonists or inhibitors, in the absence of an agonist or inhibitor, by testing whether the candidate compound results in inhibition of activation of the polypeptide or polynucleotide, as the case may be. Further, the screening methods may simply comprise the steps of mixing a candidate compound with a solution containing a polypeptide or polynucleotide of the present invention, to form a mixture, measuring BASB103 polypeptide and/or polynucleotide activity in the mixture, and comparing the BASB 103 polypeptide and/or polynucleotide activity of the mixture to a standard. Fusion proteins, such as those made from Fc portion and BASB103 as hereinbefore described, can also be used for high-throughput screening assays to identify antagonists of the polypeptide of the present invention, as well as of phylogenetically and and/or functionally related polypeptides (see D. Bennett et al., J Mol Recognition, 8:52-58 (1995); and K. Johanson et al., J Biol Chem, 270(16):9459-9471 (1995)).

The polynucleotides, polypeptides and antibodies that bind to and/or interact with a polypeptide of the present invention may also be used to configure screening methods for detecting the effect of added compounds on the production of mRNA and/or polypeptide in cells. For example, an ELISA assay may be constructed for measuring secreted or cell associated levels of polypeptide using monoclonal and polyclonal antibodies by standard methods known in the art. This can be used to discover agents which may inhibit or enhance the production of polypeptide (also called antagonist or agonist, respectively) from suitably manipulated cells or tissues.

The invention also provides a method of screening compounds to identify those which enhance (agonist) or block (antagonist) the action of BASB 103 polypeptides or polynucleotides, particularly those compounds that are bacteriostatic and/or bactericidal. The method of screening may involve high-throughput techniques. For example, to screen for agonists or antagonists, a synthetic reaction mix, a cellular compartment, such as a membrane, cell envelope or cell wall, or a preparation of any thereof, comprising BASB103 polypeptide and a labeled substrate or ligand of such polypeptide is incubated in the absence or the presence of a candidate molecule that may be a BASB 103 agonist or antagonist. The ability of the candidate molecule to agonize or antagonize the BASB 103 polypeptide is reflected in decreased binding of the labeled ligand or decreased production of product from such substrate. Molecules that bind gratuitously, *i.e.,* without inducing the effects of BASB103 polypeptide are most likely to be good antagonists. Molecules that bind well and, as the case may be, increase the rate of product production from substrate, increase signal transduction, or increase chemical channel activity are agonists. Detection of the rate or level of, as the case may be, production of product from substrate, signal transduction, or chemical channel activity may be enhanced by using a reporter system. Reporter systems that may be useful in this regard include but are not limited to colorimetric, labeled substrate converted into product, a reporter gene that is responsive to changes in BASB103 polynucleotide or polypeptide activity, and binding assays known in the art.

Another example of an assay for BASB103 agonists is a competitive assay that combines BASB103 and a potential agonist with BASB103 binding molecules, recombinant BASB103 binding molecules, natural substrates or ligands, or substrate or ligand mimetics, under appropriate conditions for a competitive inhibition assay. BASB103 can be labeled, such as by radioactivity or a colorimetric compound, such that the number of BASB103 molecules bound to a binding molecule or converted to product can be determined accurately to assess the effectiveness of the potential antagonist.

Potential antagonists include, among others, small organic molecules, peptides, polypeptides and antibodies that bind to a polynucleotide and/or polypeptide of the invention and thereby inhibit or extinguish its activity or expression. Potential antagonists also may be small organic molecules, a peptide, a polypeptide such as a closely related protein or antibody that binds the same sites on a binding molecule, such as a binding molecule, without inducing BASB103-induced activities, thereby preventing the action or expression of BASB103 polypeptides and/or polynucleotides by excluding BASB 103, polypeptides and/or polynucleotides from binding.

Potential antagonists include a small molecule that binds to and occupies the binding site of the polypeptide thereby preventing binding to cellular binding molecules, such that normal biological activity is prevented. Examples of small molecules include but are not limited to small organic molecules, peptides or peptide-like molecules. Other potential antagonists include antisense molecules (see Okano, J. Neurochem. 56: 560 (1991); OLIGODEOXYNUCLEOTIDES AS ANTISENSE INHIBITORS OF GENE EXPRESSION, CRC Press, Boca Raton, FL (1988), for a description of these molecules). Preferred potential antagonists include compounds related to and variants of BASB103.

In a further aspect, the present invention relates to genetically engineered soluble fusion proteins comprising a polypeptide of the present invention, or a fragment thereof and various portions of the constant regions of heavy or light chains of immunoglobulins of various subclasses (IgG, IgM, IgA, IgE). Preferred as an immunoglobulin is the constant part of the heavy chain of human IgG, particularly IgG1, where fusion takes place at the hinge region. In a particular embodiment, the Fc part can be removed simply by incorporation of a cleavage sequence which can be cleaved with blood clotting factor Xa. Furthermore, this invention relates to processes for the preparation of these fusion proteins by genetic engineering, and to the use thereof for drug screening, diagnosis and therapy. A further aspect of the invention also relates to polynucleotides encoding such fusion proteins. Examples of fusion protein technology can be found in International Patent Application Nos. WO94/29458 and WO94/22914.

Each of the polynucleotide sequences provided herein may be used in the discovery and development of antibacterial compounds. The encoded protein, upon expression, can be used as a target for the screening of antibacterial drugs. Additionally, the polynucleotide sequences encoding the amino terminal regions of the encoded protein or Shine-Delgarno or other translation facilitating sequences of the respective mRNA can be used to construct antisense sequences to control the expression of the coding sequence of interest.

The invention also provides the use of the polypeptide, polynucleotide, agonist or antagonist of the invention to interfere with the initial physical interaction between a pathogen or pathogens and a eukaryotic, preferably mammalian, host responsible for sequelae of infection. In particular, the molecules of the invention may be used: in the prevention of adhesion of bacteria, in particular gram positive and/or gram negative bacteria, to eukaryotic, preferably mammalian, extracellular matrix proteins on in-dwelling devices or to extracellular matrix proteins in wounds; to block bacterial adhesion between eukaryotic, preferably mammalian, extracellular matrix proteins and bacterial BASB103 proteins that mediate tissue damage and/or; to block the normal progression of pathogenesis in infections initiated other than by the implantation of in-dwelling devices or by other surgical techniques.

In accordance with yet another aspect of the invention, there are provided BASB103 agonists and antagonists, preferably bacteristatic or bactericidal agonists and antagonists.

The antagonists and agonists of the invention may be employed, for instance, to prevent, inhibit and/or treat diseases.

In a further aspect, the present invention relates to mimotopes of the polypeptide of the invention. A mimotope is a peptide sequence, sufficiently similar to the native peptide (sequentially or structurally), which is capable of being recognised by antibodies which recognise the native peptide; or is capable of raising antibodies which recognise the native peptide when coupled to a suitable carrier.

Peptide mimotopes may be designed for a particular purpose by addition, deletion or substitution of elected amino acids. Thus, the peptides may be modified for the purposes of ease of conjugation to a protein carrier. For example, it may be desirable for some chemical conjugation methods to include a terminal cysteine. In addition it may be desirable for peptides conjugated to a protein carrier to include a hydrophobic terminus distal from the conjugated terminus of the peptide, such that the free unconjugated end of the peptide remains associated with the surface of the carrier protein. Thereby presenting the peptide in a conformation which most closely resembles that of the peptide as found in the context of the whole native molecule. For example, the peptides may be altered to have an N-terminal cysteine and a C-terminal hydrophobic amidated tail. Alternatively, the addition or substitution of a D-stereoisomer form of one or more of the amino acids may be performed to create a beneficial derivative, for example to enhance stability of the peptide.

Alternatively, peptide mimotopes may be identified using antibodies which are capable themselves of binding to the polypeptides of the present invention using techniques such as phage display technology (EP 0 552 267 B1). This technique, generates a large number of peptide sequences which mimic the structure of the native peptides and are, therefore, capable of binding to anti-native peptide antibodies, but may not necessarily themselves share significant sequence homology to the native polypeptide.

## Vaccines

Another aspect of the invention relates to a method for inducing an immunological response in an individual, particularly a mammal, preferably humans, which comprises inoculating the individual with BASB103. polynucleotide and/or polypeptide, or a fragment or variant thereof, adequate to produce antibody and/ or T cell immune response to protect said individual from infection, particularly bacterial infection and most particularly *Moraxella catarrhalis* infection. Also provided are methods whereby such immunological response slows bacterial replication. Yet another aspect of the invention relates to a method of inducing immunological response in an individual which comprises delivering to such individual a nucleic acid vector, sequence or ribozyme to direct expression of BASB103 polynucleotide and/or polypeptide, or a fragment or a variant thereof, for expressing BASB 103 polynucleotide and/or polypeptide, or a fragment or a variant thereof *in vivo* in order to induce an immunological response, such as, to produce antibody and/ or T cell immune response, including, for example, cytokine-producing T cells or cytotoxic T cells, to protect said individual, preferably a human, from disease, whether that disease is already established within the individual or not. One example of administering the gene is by accelerating it into the desired cells as a coating on particles or otherwise. Such nucleic acid vector may comprise DNA, RNA, a ribozyme, a modified nucleic acid, a DNA/RNA hybrid, a DNA-protein complex or an RNA-protein complex.

A further aspect of the invention relates to an immunological composition that when introduced into an individual, preferably a human, capable of having induced within it an immunological response, induces an immunological response in such individual to a BASB 103 polynucleotide and/or polypeptide encoded therefrom, wherein the composition comprises a recombinant BASB103, polynucleotide and/or polypeptide encoded therefrom and/or comprises DNA and/or RNA which encodes and expresses an antigen of said BASB103 polynucleotide, polypeptide encoded therefrom, or other polypeptide of the invention. The immunological response may be used therapeutically or prophylactically and may take the form of antibody immunity and/or cellular immunity, such as cellular immunity arising from CTL or CD4+ T cells.

A BASB103 polypeptide or a fragment thereof may be fused with co-protein or chemical moiety which may or may not by itself produce antibodies, but which is capable of stabilizing the first protein and producing a fused or modified protein which will have antigenic and/or immunogenic properties, and preferably protective properties. Thus fused recombinant protein, preferably further comprises an antigenic co-protein, such as lipoprotein D from *Haemophilus influenzae,* Glutathione-S-transferase (GST) or beta-galactosidase, or any other relatively large co-protein which solubilizes the protein and facilitates production and purification thereof. Moreover, the co-protein may act as an adjuvant in the sense of providing a generalized stimulation of the immune system of the organism receiving the protein. The co-protein may be attached to either the amino- or carboxy-terminus of the first protein.

Provided by this invention are compositions, particularly vaccine compositions, and methods comprising the polypeptides and/or polynucleotides of the invention and immunostimulatory DNA sequences, such as those described in Sato, Y. et al. Science 273: 352 (1996).

Also, provided by this invention are methods using the described polynucleotide or particular fragments thereof, which have been shown to encode non-variable regions of bacterial cell surface proteins, in polynucleotide constructs used in such genetic immunization experiments in animal models of infection with *Moraxella catarrhalis.*
Such experiments will be particularly useful for identifying protein epitopes able to provoke a prophylactic or therapeutic immune response. It is believed that this approach will allow for the subsequent preparation of monoclonal antibodies of particular value, derived from the requisite organ of the animal successfully resisting or clearing infection, for the development of prophylactic agents or therapeutic treatments of bacterial infection, particularly *Moraxella catarrhalis* infection, in mammals, particularly humans.

The invention also includes a vaccine formulation which comprises an immunogenic recombinant polypeptide and/or polynucleotide of the invention together with a suitable carrier, such as a pharmaceutically acceptable carrier. Since the polypeptides and polynucleotides may be broken down in the stomach, each is preferably administered parenterally, including, for example, administration that is subcutaneous, intramuscular, intravenous, or intradermal. Formulations suitable for parenteral administration include aqueous and non-aqueous sterile injection solutions which may contain anti-oxidants, buffers, bacteriostatic compounds and solutes which render the formulation isotonic with the bodily fluid, preferably the blood, of the individual; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use.

The vaccine formulation of the invention may also include adjuvant systems for enhancing the immunogenicity of the formulation. Preferably the adjuvant system raises preferentially a TH1 type of response.

An immune response may be broadly distinguished into two extreme catagories, being a humoral or cell mediated immune responses (traditionally characterised by antibody and cellular effector mechanisms of protection respectively). These categories of response have been termed TH1-type responses (cell-mediated response), and TH2-type immune responses (humoral response).

Extreme TH1-type immune responses may be characterised by the generation of antigen specific, haplotype restricted cytotoxic T lymphocytes, and natural killer cell responses. In mice TH1-type responses are often characterised by the generation of antibodies of the IgG2a subtype, whilst in the human these correspond to IgGl type antibodies. TH2-type immune responses are characterised by the generation of a broad range of immunoglobulin isotypes including in mice IgGl, IgA, and IgM.

It can be considered that the driving force behind the development of these two types of immune responses are cytokines. High levels of TH1-type cytokines tend to favour the induction of cell mediated immune responses to the given antigen, whilst high levels of TH2-type cytokines tend to favour the induction of humoral immune responses to the antigen.

The distinction of TH1 and TH2-type immune responses is not absolute. In reality an individual will support an immune response which is described as being predominantly TH1 or predominantly TH2. However, it is often convenient to consider the families of cytokines in terms of that described in murine CD4 +ve T cell clones by Mosmann and Coffman *(*Mosmann, T.R. and Coffman, R.L. (1989) TH1 and TH2 cells: different patterns of lymphokine secretion lead to different functional properties. Annual Review of Immunology, 7, p145-173*).* Traditionally, TH1-type responses are associated with the production of the INF-$\gamma$ and IL-2 cytokines by T-lymphocytes. Other cytokines often directly associated with the induction of TH1-type immune responses are not produced by T-cells, such as IL-12. In contrast, TH2- type responses are associated with the secretion of IL-4, IL-5, IL-6 and IL-13.

It is known that certain vaccine adjuvants are particularly suited to the stimulation of either TH1 or TH2 - type cytokine responses. Traditionally the best indicators of the TH 1:TH2 balance of the immune response after a vaccination or infection includes direct measurement of the production of TH1 or TH2 cytokines by T lymphocytes *in vitro* after restimulation with antigen, and/or the measurement of the IgGI :IgG2a ratio of antigen specific antibody responses.

Thus, a TH1-type adjuvant is one which preferentially stimulates isolated T-cell populations to produce high levels of TH1-type cytokines when re-stimulated with antigen *in vitro,* and promotes development of both CD8+ cytotoxic T lymphocytes and antigen specific immunoglobulin responses associated with TH1-type isotype.

Adjuvants which are capable of preferential stimulation of the TH1 cell response are described in International Patent Application No. WO 94/00153 and WO 95/17209.

3 De-O-acylated monophosphoryl lipid A (3D-MPL) is one such adjuvant. This is known from GB 2220211 (Ribi). Chemically it is a mixture of 3 De-O-acylated monophosphoryl lipid A with 4, 5 or 6 acylated chains and is manufactured by Ribi Immunochem. Montana. A preferred form of 3 De-O-acylated monophosphoryl lipid A is disclosed in European Patent 0 689 454 B1 (SmithKline Beecham Biologicals SA).

Preferably, the particles of 3D-MPL are small enough to be sterile filtered through a 0.22micron membrane (European Patent number 0 689 454). 3D-MPL will be present in the range of 10$\mu$g - 100$\mu$g preferably 25-50$\mu$g per dose wherein the antigen will typically be present in a range 2-50$\mu$g per dose.

Another preferred adjuvant comprises QS21, an Hplc purified non-toxic fraction derived from the bark of Quillaja Saponaria Molina. Optionally this may be admixed with 3 De-O-acylated monophosphoryl lipid A (3D-MPL), optionally together with an carrier.

The method of production of QS21 is disclosed in US patent No. 5,057,540.

Non-reactogenic adjuvant formulations containing QS21 have been described previously (WO 96/33739). Such formulations comprising QS21 and cholesterol have been shown to be successful TH1 stimulating adjuvants when formulated together with an antigen.

Further adjuvants which are preferential stimulators of TH1 cell response include immunomodulatory oligonucleotides, for example unmethylated CpG sequences as disclosed in WO 96/02555.

Combinations of different TH1 stimulating adjuvants, such as those mentioned hereinabove, are also contemplated as providing an adjuvant which is a preferential stimulator of TH1 cell response. For example, QS21 can be formulated together with 3D-MPL. The ratio of QS21 : 3D-MPL will typically be in the order of 1 : 10 to 10 : 1; preferably 1:5 to 5 : 1 and often substantially 1 : 1. The preferred range for optimal synergy is 2.5 : 1 to 1 : 1 3D-MPL: QS21.

Preferably a carrier is also present in the vaccine composition according to the invention. The carrier may be an oil in water emulsion, or an aluminium salt, such as aluminium phosphate or aluminium hydroxide.

A preferred oil-in-water emulsion comprises a metabolisible oil, such as squalene, alpha tocopherol and Tween 80. In a particularly preferred aspect the antigens in the vaccine composition according to the invention are combined with QS21 and 3D-MPL in such an emulsion. Additionally the oil in water emulsion may contain span 85 and/or lecithin and/or tricaprylin.

Typically for human administration QS21 and 3D-MPL will be present in a vaccine in the range of 1$\mu$g - 200$\mu$g, such as 10-100$\mu$g, preferably 10$\mu$g - 50$\mu$g per dose.

Typically the oil in water will comprise from 2 to 10% squalene, from 2 to 10% alpha tocopherol and from 0.3 to 3% tween 80. Preferably the ratio of squalene: alpha tocopherol is equal to or less than 1 as this provides a more stable emulsion. Span 85 may also be present at a level of 1%. In some cases it may be advantageous that the vaccines of the present invention will further contain a stabiliser.

Non-toxic oil in water emulsions preferably contain a non-toxic oil, e.g. squalane or squalene, an emulsifier, e.g. Tween 80, in an aqueous carrier. The aqueous carrier may be, for example, phosphate buffered saline.

A particularly potent adjuvant formulation involving QS21, 3D-MPL and tocopherol in an oil in water emulsion is described in WO 95/17210.

The present invention also provides a polyvalent vaccine composition comprising a vaccine formulation of the invention in combination with other antigens, in particular antigens useful for treating cancers, autoimmune diseases and related conditions. Such a polyvalent vaccine composition may include a TH-1 inducing adjuvant as hereinbefore described.

While the invention has been described with reference to certain BASB103 polypeptides and polynucleotides, it is to be understood that this covers fragments of the naturally occurring polypeptides and polynucleotides, and similar polypeptides and polynucleotides with additions, deletions or substitutions which do not substantially affect the immunogenic properties of the recombinant polypeptides or polynucleotides.

## Compositions, kits and administration

In a further aspect of the invention there are provided compositions comprising a BASB103, polynucleotide and/or a BASB103 polypeptide for administration to a cell or to a multicellular organism.

The invention also relates to compositions comprising a polynucleotide and/or a polypeptides discussed herein or their agonists or antagonists. The polypeptides and polynucleotides of the invention may be employed in combination with a non-sterile or sterile carrier or carriers for use with cells, tissues or organisms, such as a pharmaceutical carrier suitable for administration to an individual. Such compositions comprise, for instance, a media additive or a therapeutically effective amount of a polypeptide and/or polynucleotide of the invention and a pharmaceutically acceptable carrier or excipient. Such carriers may include, but are not limited to, saline, buffered saline, dextrose, water, glycerol, ethanol and combinations thereof. The formulation should suit the mode of administration. The invention further relates to diagnostic and pharmaceutical packs and kits comprising one or more containers filled with one or more of the ingredients of the aforementioned compositions of the invention.

Polypeptides, polynucleotides and other compounds of the invention may be employed alone or in conjunction with other compounds, such as therapeutic compounds.

The pharmaceutical compositions may be administered in any effective, convenient manner including, for instance, administration by topical, oral, anal, vaginal, intravenous, intraperitoneal, intramuscular, subcutaneous, intranasal or intradermal routes among others.

In therapy or as a prophylactic, the active agent may be administered to an individual as an injectable composition, for example as a sterile aqueous dispersion, preferably isotonic.

In a further aspect, the present invention provides for pharmaceutical compositions comprising a therapeutically effective amount of a polypeptide and/or polynucleotide, such as the soluble form of a polypeptide and/or polynucleotide of the present invention, agonist or antagonist peptide or small molecule compound, in combination with a pharmaceutically acceptable carrier or excipient. Such carriers include, but are not limited to, saline, buffered saline, dextrose, water, glycerol, ethanol, and combinations thereof. The invention further relates to pharmaceutical packs and kits comprising one or more containers filled with one or more of the ingredients of the aforementioned compositions of the invention. Polypeptides, polynucleotides and other compounds of the present invention may be employed alone or in conjunction with other compounds, such as therapeutic compounds.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a polypeptide or other compounds of the present invention can be formulated in an enteric or an encapsulated formulation, oral administration may also be possible. Administration of these compounds may also be topical and/or localized, in the form of salves, pastes, gels, solutions, powders and the like.

For administration to mammals, and particularly humans, it is expected that the daily dosage level of the active agent will be from 0.01 mg/kg to 10 mg/kg, typically around 1 mg/kg. The physician in any event will determine the actual dosage which will be most suitable for an individual and will vary with the age, weight and response of the particular individual. The above dosages are exemplary of the average case. There can, of course, be individual instances where higher or lower dosage ranges are merited, and such are within the scope of this invention.

The dosage range required depends on the choice of peptide, the route of administration, the nature of the formulation, the nature of the subject's condition, and the judgment of the attending practitioner. Suitable dosages, however, are in the range of 0.1-100 μg/kg of subject.

A vaccine composition is conveniently in injectable form. Conventional adjuvants may be employed to enhance the immune response. A suitable unit dose for vaccination is 0.5-5 microgram/kg of antigen, and such dose is preferably administered 1-3 times and with an interval of 1-3 weeks. With the indicated dose range, no adverse toxicological effects will be observed with the compounds of the invention which would preclude their administration to suitable individuals.

Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

## Sequence Databases, Sequences in a Tangible Medium, and Algorithms

Polynucleotide and polypeptide sequences form a valuable information resource with which to determine their 2- and 3-dimensional structures as well as to identify further sequences of similar homology. These approaches are most easily facilitated by storing the sequence in a computer readable medium and then using the stored data in a known macromolecular structure program or to search a sequence database using well known searching tools, such as the GCG program package.

Also provided by the invention are methods for the analysis of character sequences or strings, particularly genetic sequences or encoded protein sequences. Preferred methods of sequence analysis include, for example, methods of sequence homology analysis, such as identity and similarity analysis, DNA, RNA and protein structure analysis, sequence assembly, cladistic analysis, sequence motif analysis, open reading frame determination, nucleic acid base calling, codon usage analysis, nucleic acid base trimming, and sequencing chromatogram peak analysis.

A computer based method is provided for performing homology identification. This method comprises the steps of: providing a first polynucleotide sequence comprising the sequence of a polynucleotide of the invention in a computer readable medium: and comparing said first polynucleotide sequence to at least one second polynucleotide or polypeptide sequence to identify homology.

A computer based method is also provided for performing homology identification, said method comprising the steps of: providing a first polypeptide sequence comprising the sequence of a polypeptide of the invention in a computer readable medium; and comparing said first polypeptide sequence to at least one second polynucleotide or polypeptide sequence to identify homology.

## DEFINITIONS

"Identity," as known in the art, is a relationship between two or more polypeptide sequences or two or more polynucleotide sequences, as the case may be, as determined by comparing the sequences. In the art, "identity" also means the degree of sequence relatedness between polypeptide or polynucleotide sequences, as the case may be, as determined by the match between strings of such sequences. "Identity" can be readily calculated by known methods, including but not limited to those described in (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988). Methods to determine identity are designed to give the largest match between the sequences tested. Moreover, methods to determine identity are codified in publicly available computer programs. Computer program methods to determine identity between two sequences include, but are not limited to, the GAP program in the GCG program package (Devereux, J., et al., Nucleic Acids Research 12(1): 387 (1984)), BLASTP, BLASTN (Altschul, S.F. et al., J Molec. Biol. 215: 403-410 (1990), and FASTA( Pearson and Lipman Proc. Natl. Acad. Sci. USA 85; 2444-2448 (1988). The BLAST family of programs is publicly available from NCBI and other sources *(BLAST Manual,* Altschul, S., *et al.,* NCBI NLM NIH Bethesda, MD 20894; Altschul, S., et al., J Mol. Biol. 215: 403-410 (1990). The well known Smith Waterman algorithm may also be used to determine identity.

Parameters for polypeptide sequence comparison include the following:

Algorithm: Needleman and Wunsch, J. Mol Biol. 48: 443-453 (1970)
Comparison matrix: BLOSSUM62 from Henikoff and Henikoff, Proc. Natl. Acad. Sci. USA. 89:10915-10919 (1992)
Gap Penalty: 8
Gap Length Penalty: 2
A program useful with these parameters is publicly available as the "gap" program from Genetics Computer Group, Madison WI. The aforementioned parameters are the default parameters for peptide comparisons (along with no penalty for end gaps).

Parameters for polynucleotide comparison include the following:

Algorithm: Needleman and Wunsch, J. Mol Biol. 48: 443-453 (1970)
Comparison matrix: matches = +10, mismatch = 0
Gap Penalty: 50
Gap Length Penalty: 3
Available as: The "gap" program from Genetics Computer Group, Madison WI. These are the default parameters for nucleic acid comparisons.

A preferred meaning for "identity" for polynucleotides and polypeptides, as the case may be, are provided in (1) and (2) below.

(1) Polynucleotide embodiments further include an isolated polynucleotide comprising a polynucleotide sequence having at least a 50, 60, 70, 80, 85, 90, 95, 97 or 100% identity to the reference sequence of SEQ ID NO:1, wherein said polynucleotide sequence may be identical to the reference sequence of SEQ ID NO:1 or may include up to a certain integer number of nucleotide alterations as compared to the reference sequence, wherein said alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence, and wherein said number of nucleotide alterations is determined by multiplying the total number of nucleotides in SEQ ID NO:1 by the integer defining the percent identity divided by 100 and then subtracting that product from said total number of nucleotides in SEQ ID NO:1, or:

$$n_n \leq x_n - (x_n \bullet y),$$

wherein $n_n$ is the number of nucleotide alterations, $x_n$ is the total number of nucleotides in SEQ ID NO:1, y is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $x_n$ and y is rounded down to the nearest integer prior to subtracting it from $x_n$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.

By way of example, a polynucleotide sequence of the present invention may be identical to the reference sequence of SEQ ID NO:1, that is it may be 100% identical, or it may include up to a certain integer number of nucleic acid alterations as compared to the reference sequence such that the percent identity is less than 100% identity. Such alterations are selected from the group consisting of at least one nucleic acid deletion, substitution, including transition and transversion, or insertion, and wherein said alterations may occur at the 5' or 3' terminal positions of the reference polynucleotide sequence or anywhere between those terminal positions, interspersed either individually among the nucleic acids in the reference sequence or in one or more contiguous groups within the reference sequence. The number of nucleic acid alterations for a given percent identity is determined by multiplying the total number of nucleic acids in SEQ ID NO:1 by the integer defining the percent identity divided by 100 and then subtracting that product from said total number of nucleic acids in SEQ ID NO:1, or:

$$n_n \leq x_n - (x_n \bullet y),$$

wherein $n_n$ is the number of nucleic acid alterations, $x_n$ is the total number of nucleic acids in SEQ ID NO:1, y is, for instance 0.70 for 70%, 0.80 for 80%, 0.85 for 85% etc. • is the symbol for the multiplication operator, and wherein any non-integer product of $x_n$ and y is rounded down to the nearest integer prior to subtracting it from $x_n$.

(2) Polypeptide embodiments further include an isolated polypeptide comprising a polypeptide having at least a 50,60, 70, 80, 85, 90, 95. 97 or 100% identity to a polypeptide reference sequence of SEQ ID NO:2, wherein said polypeptide sequence may be identical to the reference sequence of SEQ ID NO:2 or may include up to a certain integer number of amino acid alterations as compared to the reference sequence, wherein said alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence, and wherein said number of amino acid alterations is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $\mathbf{n}_a$ is the number of amino acid alterations, $\mathbf{x}_a$ is the total number of amino acids in SEQ ID NO:2, y is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $\mathbf{x}_a$ and y is rounded down to the nearest integer prior to subtracting it from $\mathbf{x}_a$. ,

By way of example, a polypeptide sequence of the present invention may be identical to the reference sequence of SEQ ID NO:2, that is it may be 100% identical, or it may include up to a certain integer number of amino acid alterations as compared to the reference sequence such that the percent identity is less than 100% identity. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $n_a$ is the number of amino acid alterations, $\mathbf{x}_a$ is the total number of amino acids in SEQ ID NO:2, y is, for instance 0.70 for 70%, 0.80 for 80%, 0.85 for 85% etc., and • is the symbol for the multiplication operator, and wherein any non-integer product of $\mathbf{x}_a$ and y is rounded down to the nearest integer prior to subtracting it from $\mathbf{x}_a$.

"Individual(s)," when used herein with reference to an organism, means a multicellular eukaryote, including, but not limited to a metazoan, a mammal, an ovid, a bovid, a simian, a primate, and a human.

"Isolated" means altered "by the hand of man" from its natural state, *i.e.,* if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide(s)" generally refers to any polyribonucleotide or polydeoxyribonucleotide, which may be unmodified RNA or DNA or modified RNA or DNA including single and double-stranded regions.

"Variant" refers to a polynucleotide or polypeptide that differs from a reference polynucleotide or polypeptide, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Disease(s)" means any disease caused by or related to infection by a bacteria, including, for example, otitis media in infants and children, pneumonia in elderlies, sinusitis, nosocomial infections and invasive diseases, chronic otitis media with hearing loss, fluid accumulation in the middle ear, auditive nerve damage, delayed speech learning, infection of the upper respiratory tract and inflammation of the middle ear.

EXAMPLES:

The examples below are carried out using standard techniques, which are well known and routine to those of skill in the art, except where otherwise described in detail. The examples are illustrative, but do not limit the invention.

**Example 1: DNA sequencing of the BASB103, BASB104, BASB105, BASB106, BASB107 and BASB108 gene from *Moraxella catarrhalis* strain ATCC 43617.**

The BASB103 gene of SEQ ID NO:1 and are from *Moraxella catarrhalis* strain ATCC 43617. The translation of the BASB 103 polynucleotide sequences are shown in SEQ ID NO:2.

**Deposited materials**

A deposit containing a *Moraxella catarrhalis* Catlin strain has been deposited with the American Type Culture Collection (herein "ATCC") on June 21, 1997 and assigned deposit number 43617. The deposit was described as *Branhamella catarrhalis* (Frosch and Kolle) and is a freeze-dried, 1.5-2.9 kb insert library constructed from M. catarrhalis isolate obtained from a transtracheal aspirate of a coal miner with chronic bronchitits. The deposit is described in Antimicrob. Agents Chemother. 21: 506-508 (1982).

The *Moraxella catarrhalis* strain deposit is referred to herein as "the deposited strain" or as "the DNA of the deposited strain."

The deposited strain contains a full length BASB 103, BASB 104, BASB 105, BASB 106, BASB 107 and BASB 108 gene.

A deposit of the vector pMC-D15 consisting of *Moraxella catarrhalis* DNA inserted in pQE30 has been deposited with the American Type Culture Collection (ATCC) on February 12 1999 and assigned deposit number 207105.

The sequence of the polynucleotides contained in the deposited strain / clone, as well as the amino acid sequence of any polypeptide encoded thereby, are controlling in the event of any conflict with any description of sequences herein.

The deposit of the deposited strains have been made under the terms of the Budapest Treaty on the International Recognition of the Deposit of Micro-organisms for Purposes of Patent Procedure. The deposited strains will be irrevocably and without restriction or condition released to the public upon the issuance of a patent. The deposited strains are provided merely as convenience to those of skill in the art and are not an admission that a deposit is required for enablement, such as that required under 35 U.S.C. § 112.

**SEQUENCE INFORMATION**

**BASB103, BASB104, BASB105, BASB106, BASB107 and BASB108 Polynucleotide and Polypeptide Sequences**

**SEQ ID NO:1**

***Moraxella calarrhalis* BASB103 polynucleotide sequence from strain ATCC43617**

```
ATGAAAGCTGTTAAATTATCTGTAGTTTCTGCTGCAGTTCTACTGTCAACTTCTGCAATG
GCAAATGTTGTTACCAATACTGGTGCGACCGTTGTTGACGGTACTCGTACCATCTTTAGT
ACACTGGTTAGACCTGCGGCTGTGAGTGCTGAGGTTGGTACTTTGGGTTATGGTGCTAAT
ATTGGCTGGGCGGTGAATGACACTGTTGAGCTACAAGCAGGTTGGGCTGGTGGCAATATC
GCAAAATCCATCAATGATAACTTTAATGCCAATGGCGTTAACTATCAGCTTGAAACTGAT
TTTTCTAACCCATACTTAGGTGCTCAAGTGCGTCCTGCTGCCAACTGGTTCACCGTTGGG
ACAGGTATCATCGTACCTAAGAACGACATCAAAGTCCGCTCAAATAACACAACCGATGGT
GTTTTCCGTGTTGATGGTAAAGACTACAAACAAAGTGATGTAGGTCAGCTTACTGGTAAG
CTTGAGCATCGTAACAAATTAGCACCTTATTTGACACTGGGTTTCCGCCCAAATCTACAC
AGTAACTGGGGTGTATTTGGTGAAGTTGGTGCTGCCTATTTGGGTAAAGTAGATGCTACT
GTTGATGCACAAAATCCAACCAAATCAGTAACTGCTACTGATAGCAAGACCCAAGCGACT
GTCATTAAAATTGCAAAACAAGCAGAGCGTGACATCGAAGATAAAAAATATGCCAACTGG
TTCCCAATCGCTAAAGTTGGTGTTACTTACCGTTTCTAA
```

**SEQ ID NO:2**

***Moraxella catarrhalis* BASB103 polypeptide sequence deduced from the polynucleotide of SEQ ID NO:1**

```
MKAVKLSVVSAAVLLSTSAMANVVTNTGATVVDGTRTIFSTLVRPAAVSAEVGTLGYGAN
IGWAVNDTVELQAGWAGGNIAKSINDNFNANGVNYQLETDFSNPYLGAQVRPAANWFTVG
TGIIVPKNDIKVRSNNTTDGVFRVDGKDYKQSDVGQLTGKLEHRNKLAPYLTLGFRPNLH
SNWGVFGEVGAAYLGKVDATVDAQNPTKSVTATDSKTQATVIKIAKQAERDIEDKKYANW
FPIAKVGVTYRF
```

**SEQ ID NO:3**

***Moraxella catarrhalis* BASB104 polynucleotide sequence from strain ATCC43617**

```
ATGATTGATCATCTTTCTTTTAAAACGACTAAGGCCAAAAGTCTTCATACTCAAAATATC
GCCCAAAGAACGCTCAAAGCGGTGGCTCATACATACCGCCCTTTATCAATGGCGATTGCA
CTGATGGGCTTAAGCAGCATCGCCACCGCCCAAGAATTTAGCCAAACCGTATTTTTTGGT
GATAGCCTAACTGACACAGGACGCTTGGTGCAGATTGGCAAAAACAGTCTGGCGTCTTCT
GTTTTTAATCAAGCCCAACCATCGTTTACGACCAATACCGATCCAGTTTGGTCAAGCATA
TTGGCAAAATCTTATGGACATACCGCCGATGCCAATGATGGAACAACCTTAACTGGTACA
AACTATGCCGTTGGTGGTGCAAGAACTAAAGAAGATGTGGTCAAAAATGCACCTGTTCCT
TTTTTCACCATTCCTTTATTTACCATCCCATCAGCACAAACCCAAATCAATCGCTACCTG
ACCTTAAATAATCATCAAGCCGACCCCAAAGCATTGTATACTGTTTGGACAGGTGCCAAT
GATTTGTTTGAGGCAGCCAAAGCACCAACCCAGTTGCAAGCAGCCGAAATCATTACCACC
GCTGCCAATGACCAAGCCAATTTGGTTGGGCAGCTTGGGCAAGCAGGTGCAAAACACATT
TTAGTACCCAGTCTTCCTGATGTTGGCGTCACGCCAGAATACGCCCAAGATCCGACCAAA
TCTGCCACAGCATCATTGTCTGCTCATATCTACAACCAAACTTTATATCAAAGTTTAAAC
AACCAAACCACCAATGTCATTGCTGCCAATACCTTTGCCCTACTCAAAGAGGCGGTTAGT

AATCCAGCAGGATTTGGGTTTAGTAATGTGACCGAACCTGCTTGCCAAAACCTAGATGCC
AATTTATCATCGTTAGCATGTAAACCAAGCGACTGGCAACAAACCGCTGCGAATGCTAAT
GAAACTTATGCGTTTGCTGATAAAATTCACCCTTCAGGGCGTACGCATCGCATTTTGGCA
CAATATTATCGCTCTATTATCGATAGCCCCAACCAAATGGGTCAACTACCCCAGCATCTG
ATTAAACATGGTCAACAAAGCCAGCAACAATTGGTACGCCGCTTAGACAGTCTTAACAAT
CATCAACAATCGCTATGGATTGATGGCAGCATCAGCAATCAATCGCTTGATACACGCAAT
AAAATAGACCGCCCTACCATCAAACTTGGACTTGATATTGCACGACCTAACCAGCATACA
GGGGCATATTTAACCTACCAACAACAAGATTATCATCTAAGCGATTCTATAACCGCTGAT
GTCAAACAAACAGGCATTGGTTTGTATCATCGCCATGATTTTGATCACTTACGCATCAGT
GCCAACCTTGGGGTTGACCATTTAAGTACACAAAGCTTACGCCAAATCATGTGGGACGGT
GAGAATCGCAACCATCAAGCACATGCCACAGGTAAGCGTCGATATGCAAGTGTACAAGGC
AGCTATGGATTTACCCAAAACAATGTTACCTATCGTCCTTATGTCGGTATTCATGCTCAA
GATATCAAGCAGAATCGCTTCTTTGAAAATCAACCAAATCTATCAACTGCTCTAAGTTTT
AAGCTGCCTGACCATCAGTCTTTGCAAGCTGATATTGGCGTTAATATTGATTATGCCATG
AATGATAAACTAAACCTTTTGGCAGGATTGGGTTATCAGCATGAGTTTAAAGACAATAAC
AAAACTGTTGAGACAGCTGTATTATCCAACCGTGATTATCATCGCAGCTTTGTCACAACC
GTGCCCTTTGATAAAAAGCACACAACGCACGCACATTTAGGTGCAACACTTGCTTTGGGT
AATAACACTCATTTGAATGCTGGCATACAAGCCAATCACCAAGATCATGATACAAAAGTT
GGTGGATTTGTGGGTGCACAAATGGCATTTTAA
```

**SEQ ID NO:4**

***Morarella catarrhalis*** **BASB104 polypeptide sequence deduced from the polynucleotide of SEQ ID NO:3**

MIDHLSFKTTKAKSLHTQNIAQRTLKAVAHTYRPLSMAIALMGLSSIATAQEFSQTVFFG
DSLTDTGRLVQIGKNSLASSVFNQAQPSFTTNTDPVWSSILAKSYGHTADANDGTTLTGT
NYAVGGARTKEDVVKNAPVPFFTIPLFTIPSAQTQINRYLTLNNHQADPKALYTVWTGAN
DLFEAAKAPTQLQAAEIITTAANDQANLVGQLGQAGAKHILVPSLPDVGVTPEYAQDPTK
SATASLSAHIYNQTLYQSLNNQTTNVIAANTFALLKEAVSNPAGFGFSNVTEPACQNLDA
NLSSLACKPSDWQQTAANANETYAFADKIHPSGRTHRILAQYYRSIIDSPNQMGQLPQHL
IKHGQQSQQQLVRRLDSLNNHQQSLWIDGSISNQSLDTRNKIDRPTIKLGLDIARPNQHT
GAYLTYQQQDYHLSDSITADVKQTGIGLYHRHDFDHLRISANLGVDHLSTQSLRQIMWDG
ENRNHQAHATGKRRYASVQGSYGFTQNNVTYRPYVGIHAQDIKQNRFFENQPNLSTALSF
KLPDHQSLQADIGVNIDYAMNDKLNLLAGLGYQHEFKDNNKTVETAVLSNRDYHRSFVTT
VPFDKKHTTHAHLGATLALGNNTHLNAGIQANHQDHDTKVGGFVGAQMAF

**SEQ ID NO:5**

***Moraxella catarrhalis*** **BASB105 polynucleotide sequence from strain ATCC43617**

ATGAAAAAAAACTTCCACACAGCTTGGGCTACTTGCCGTCAGCGTTTCGTTGATTATGGCA
AGTTTACCTGCACATGCTGTTTATCTTGACCGTAACTTAAGAGATGGTCTGAAATTTGGT
ATCAGTGGTTCTGTCAATCCCAGCCTTAGCGTCAATTCAAGCACTTTTACTTATTTGGGT
GACTCATCAGTATATGGCAATAATGCCACTTTAGAGCGTATGCTACAAGACCAAGACAGA
CAAGACAGTGATGAGCGAGCAAGACTCAATGGATTTGGTGGTGCTTCTGTTTATCTGGGT
GCCCAAAAATACCTAACTCGGGATATTACTTTATTTGGTAATGTTGGTTTGTATGCACCA
GCAAGCAAAGGTCAAAGAGCTGCATATGGCTATGGCGTGAATCTTGCCACCAAATATGGC
AGTATCGGTATTAATACTGATAATGAATTTAGTGCTGGTGCTGGTACGCCCAGCGGAATT
TATAATTTGGTTGATGGCTCAAACGAGTACAGCACTGCCATATCGGTTAGTACCAGCTAT
ATCCCTAAGTTTAAGTTTAGTGCATACCATGCATTGCCTGGCTCACCTGATACACGGTCG
GTAAGTAGCCACGAAAACTACTATATCCAAAAAGCACAAGGTCTTTCTGCATCTTATAGC
CATCCTATTAGCCCAAATCAAACCCTGTCTGTTGGCACGGCTTATAGCAAAAGCCAAAGG
CACAAAGATTTTTTTAGCGATACCGCCTATAATAACAAAACAGCGTCCACTGTGGGGCTA

AGTTACCGCCAAGGAGATTGGAGTATTAGCGGTAATGTTGGTCAAGCCAAAGAAAATTTA
CACGGTGCGATCATTGATGATATTACCACAAAAGCTTTTGGGACAAAAATTAGCTACAAA
GTAACCCCAAGAATTTCTGTTTCTGGGACTTATGGACAAAAAACCACCGATAAAAATACC
AAGCCCAACAAACGCTTGGATATACCAAATATCATCGCACAACGAGGAGGTAATATTTCA
AGCCGTGTGCATGAAAGTTGGTTTTTTGATAAAACCAAACAAAAAACCTATGGTCTGAGT
GCAAGTTATTATATCTATGGCGGCATATCGATTTCTGCTTCGATGAACCAAACACGCACC
ACCAACTTTACCGAAGAAGGTGCTTATAGTGAGCGTAAGAATAACAGCTATCGCATTTCA
ACTGGCTTTTCATTTTAA

**SEQ ID NO:6**

***Moraxella catarrhalis* BASB105 polypeptide sequence deduced from the polynucleotide of SEQ ID NO:5**

```
MKKTSTQLGLLAVSVSLIMASLPAHAVYLDRNLRDGLKFGISGSVNPSLSVNSSTFTYLG
DSSVYGNNATLERMLQDQDRQDSDERARLNGFGGASVYLGAQKYLTRDITLFGNVGLYAP
ASKGQRAAYGYGVNLATKYGSIGINTDNEFSAGAGTPSGIYNLVDGSNEYSTAISVSTSY
IPKFKFSAYHALPGSPDTRSVSSHENYYIQKAQGLSASYSHPISPNQTLSVGTAYSKSQR
HKDFFSDTAYNNKTASTVGLSYRQGDWSISGNVGQAKENLHGAIIDDITTKAFGTKISYK
VTPRISVSGTYGQKTTDKNTKPNKRLDIPNIIAQRGGNISSRVHESWFFDKTKQKTYGLS
ASYYIYGGISISASMNQTRTTNFTEEGAYSERKNNSYRISTGFSF
```

**SEQ ID NO:7**

***Moraxella catarrhalis* BASB106 polynucleotide sequence from strain ATCC43617**

```
ATGAAACCATCAATCATCAAAAACCCATTAAAGCTTTTGACCGCCATGATTTTGTTAAAC
GCAATGCCTAGCCATGCGATTTATAATCTGTATAAAAGCGGTGATTTTAGCTTCGATGTG
CATGGTGAGATTAACACTTATGGGCAAAAAAATAGCCAAAAATATACCTACCTTTATCCA
GAAACAGGCTGGGCAAGTGTTAACAATGACTACGAAGCCATCACCAAAGGTGCTTATGAG
CAAAGAAGTGATCGCCGTGTACGCTTGGGGCAAGATACAGGTGCTTCTTGGACGGAGTTT
CGTGCATCTAGAAAACTAAGAGATGGCTGGCGTGTCAGTGGGGCAATTGGTTTTGGTTAT
TATGATAGCGGTACAGGTATGTACCTAAATAGTGCTAATATGGCATTTGATAAAAAAGAT
TTAGGTTCTTTATCATTGGGTCGTCAATATCTGCACACAGGCTATGTCACACGCACCAAT
ACCTATACACCACTTGAAACTTTTGGGGAAAATAGCATCCGCTTGGATTATACTGCCATT
AAAGGATTGCATGCCAGTGGCTATTATAGCCTGCCAGCTTCAAGCGATGTTCGTAAAAAG
AAGAATGGGCAAGAGGTAGAGGGTTTTGGTGCGTCTGTTAGCTACCTTCATCCACTTGAT
GATCATCAAACGGTGCGTGTTGCATTGGGCTATAGTGATAGCCGTCAAAACGCAAAAACC
ACAGGTAGAGACAGTAATTTTTATGCTACCAAGAGCCAAGGTACGGCAGCTTCGGTTGAA
TATCGCAACAATAAACTGCTATTGGCGGCAGATATTGGGCAAAAAAATGAACAAATCAAT
GGTACTGTTACCGATAAATCTAAGGGCAACTACATGGGCGTTAAGGTAGGTTATGAAATC
ACGCCTCGCCTAACCATGACGGCAGGCTTTGGTAAAAAAGTTGCTGAACGCACCCAAAAA
AGCGGTGCAGCACTGATACATGCCTACCAAAAAGACTTGTGTGTTGCTGATGCCAATGAT
TCATGCCATAATTTTGTTCAGGCTTATGAAACAGCGTTATTTGATAAGATTGATTCAAAA
CGCTCTTATGTGCGTGCCGATTATTATCTAAGAGAAAATGTACGCCTATACGGCCGTATT
GATGACGAAAAAATTACCAATCAGTTGGGTAATAAAGACTTTAGCAAGCTTCACAATACA
GGCTATCGTGCTGGCGTGTCATTCATTTTCTAA
```

**SEQ ID NO:8**

***Moraxelia catarrhalis* BASB106 polypeptide sequence deduced from the polynucleotide of SEQ ID NO:7**

MKPSIIKNPLKLLTAMILLNAMPSHAIYNLYKSGDFSFDVHGEINTYGQKNSQKYTYLYP
ETGWASVNNDYEAITKGAYEQRSDRRVRLGQDTGASWTEFRASRKLRDGWRVSGAIGFGY
YDSGTGMYLNSANMAFDKKDLGSLSLGRQYLHTGYVTRTNTYTPLETFGENSIRLDYTAI
KGLHASGYYSLPASSDVRKKKNGQEVEGFGASVSYLHPLDDHQTVRVALGYSDSRQNAKT
TGRDSNFYATKSQGTAASVEYRNNKLLLAADIGQKNEQINGTVTDKSKGNYMGVKVGYEI
TPRLTMTAGFGKKVAERTQKSGAALIHAYQKDLCVADANDSCHNFVQAYETALFDKIDSK
RSYVRADYYLRENVRLYGRIDDEKITNQLGNKDFSKLHNTGYRAGVSFIF

**SEQ ID NO:9**

***Moraxella catarrhalis* BASB107 polynucleotide sequence from strain ATCC43617**

ATGAAGGTTACCATGATAAAAAAACCGCTTGCCTGTGCCATATTGGCAACTTTTTCAATG
CCAATGCTGGCAGAGGCGAATTTAAAGGATAAGCCAACCGTCATTTTAGATGGCGTTTCG
ATCACCTCTTTAGCTGACCAAAATACAGAGTTTGGCGTTAATCATTCAAAAACAGTCAGT
GGCATCACAGTTTCAAAAGAGCAACTACAACAACGAGCAACCACCCTAGGCGATGCCTTG
GCAGGTGAGCTTGGCGTTCATTCTAACCATTTTGGGGGCGGTGCCTCAGCCCCCATCATT
CGTGGGCAGGAGGGTAAACGCCTGAAAATCCTACAAAACGGTTCAGAGGTTGTGGACATG
TCTGGGTTGTCGCCAGACCATGCCATAGCGGTGGACACCACACTGGCAAAACAGGTGGAG
ATTGTGCGAGGCTCTGGTGCCTTGTTGTACGCCTCTGGCAACTCAGCAGGCGTGGTCAAT
GTCGTTGATGACAAAAATACCCAGCAAATTGCCCAGCAAATTACAAGGTGATGTGACGGTG
CGTCTTAGCAGTGCCAACCGTGAAAAAATTAATCACCGCCAGTGCCGAAGCCCCACTGGGA
GAGCATGTGGCAGTGCGTGTTGCAGGGCTGTCCAAACAAGCAGCAGACTATAAAACGCCA
CGCTTTGACCGCCATGTCTTTAACAAAAAACATGAAGATGATAACACTCAGCCAGAATTC
ATCTATAAAGACACCTTAAAGCATCTGCCAGACAGCCATGCCAAATCAAACGCAGGAACG
CTTGGCGTGTCATGGGTTGGCAATCAAGGCTTTTTGGGGGCATCGGTGAGCTTACGCCGA
GACAAATATGGCCTGCCCAACCATTCACATGAATATGAAGAATGTAGCGTGCATGGCATT
TCTCAGTCCGCCTTACAATACAAGCCATATTTGCGTTTGTATCCTTTTTTGATGGAAAAT
GATGACTTAGAGTTTGACAATGCAGGGCTTGAATGCCATACACATGATGACCACGACCAC
GAGCACGACCATGCTCATGACCACGAGCACGACCACGAGCACGACCATGGCAAACCTTGG
ATTGATTTGAAAATGAAGCGTTATGATGTGCAAGGGCAAATCAATGCGCCGTTTGCTGGC
ATTGATAAAATCCGAGCCAGCATGGGCAAAGTGGATTATCATCATGATGAGATAGATGGG
GGTGAGAAGACCAGCTTTTTTGATAATCAAGCCAATGTGTGGCGTCTGGAAGCCTCACAT
ACCCCCATTCATACGCCGATGGGCAAGTTTAGCGGGGTGTTTGGGGTAGGTTATCTCACC
TCAAAAAACAGCGGACTTGTGCCACCTCGTTATGAAGATGGCAATAAACAAGACACCCAA
AACATCTTGCACAATAATAAAACCAAAACAGGCAGTGTGTTTTGGTTTGAAGAATACAAA
CCCAATGACAAGCTGACCGTTGACGCCGCCGCTCGCATTGAGAAACAAACCATCACCATG
GATTATGATAAAGACGCCATTTATCAGAGCTTAAACTTAGGCTTAGCAACCGCTCATGAA
CCAGACATACGCTTTAAACGATTGCTGGACAGCGGTACTTTAAACCCCAAAAAACAAACC
GCACGCTCTTATGCTGTTGGGACGCATTTACAATTAACGCCCAAACATAAATTATCGCTG
AATCTGTCGCATCAAGAACGCCTGCCAAATGCTCAGGAATTGTATGCTCACGGCATGCAC
CTTGCCACCAACTCGTTTGAAATTGGCAACCGCTTTTTAAACAAAGAAAAATCCAACAAC
ATTGATTTGGGGCTGACATTTCAAGGTGATAAATGGGATTATCGTCTTGGGGGCTATCAT
TATGATTTTGATAACTATGTGTTTTTACAAACATTGTCGCAGTATAAGCAAGGTTTGCGT
GGCATGCGTCATGATAAAGACTTAAAAACCGCACGCTATGAACAAGCAGCGGCGAAATTT
TATGGATTTGATGTCAACATCGGTTATCAGATTAATGATGTATATCATGTGGCGTTATTT

GGTGATTATATTCGTGGCAAGCTCACCAATTTGCCTGACAAAAAGGGCAGAACCGATGCG
TATGGCAACCGTCCTCTCATCAAACAGCCAGACAGTCATACGCCAAGACTGCCACCAAAA
CGCCTTGGCATGAAATTAACCGCCAATGTTAATGCAAATTGGTCAGGGTTTTTGGAATAT
CGCCATACCTTTAAACAAGATAAATTGGCGAATTTTGAACGCCCAACCCCAGCTCATAAC
TTGGTGAATTTGGGGCTTAACTATCAGCACAAGCCAAGCCATCAAGCAGGCTCGGTTCAG
GTATTTTTTAATGCTAACAATCTATTAAACGATAAAGTCTTTGCTCATGAGACATTTTTC
CCAGACATGCCACAAATGGGGCGAAACTTTATGCTCGGGGCAAATTTCAAATTTTGA

**SEQ ID NO:10**

***Moraxella catarrhalis* BASB107 polypeptide sequence deduced from the polynucleotide of SEQ ID NO:9**

MKVTMIKKPLACAILATFSMPMLAEANLKDKPTVILDGVSITSLADQNTEFGVNHSKTVS
GITVSKEQLQQRATTLGDALAGELGVHSNHFGGGASAPIIRGQEGKRLKILQNGSEVVDM
SGLSPDHAIAVDTTLAKQVEIVRGSGALLYASGNSAGVVNVVDDKIPSKLPSKLQGDVTV
RLSSANREKLITASAEAPLGEHVAVRVAGLSKQAADYKTPRFDRHVFNKKHEDDNTQPEF
IYKDTLKHLPDSHAKSNAGTLGVSWVGNQGFLGASVSLRRDKYGLPNHSHEYEECSVHGI
SQSALQYKPYLRLYPFLMENDDLEFDNAGLECHTHDDHDHEHDHAHDHEHDHEHDHGKPW
IDLKMKRYDVQGQINAPFAGIDKIRASMGKVDYHHDEIDGGEKTSFFDNQANVWRLEASH
TPIHTPMGKFSGVFGVGYLTSKNSGLVPPRYEDGNKQDTQNILHNNKTKTGSVFWFEEYK
PNDKLTVDAAARIEKQTITMDYDKDAIYQSLNLGLATAHEPDIRFKRLLDSGTLNPKKQT
ARSYAVGTHLQLTPKHKLSLNLSHQERLPNAQELYAHGMHLATNSFEIGNRFLNKEKSNN
IDLGLTFQGDKWDYRLGGYHYDFDNYVFLQTLSQYKQGLRGMRHDKDLKTARYEQAAAKF
YGFDVNIGYQINDVYHVALFGDYIRGKLTNLPDKKGRTDAYGNRPLIKQPDSHTPRLPPK
RLGMKLTANVNANWSGFLEYRHTFKQDKLANFERPTPAHNLVNLGLNYQHKPSHQAGSVQ
VFFNANNLLNDKVFAHETFFPDMPQMGRNFMLGANFKF

**SEQ ID NO:11**

***Moraxella catarrhalis* BASB108 polynucleotide sequence from strain ATCC43617**

ATGATAAAAAAACCACTTGTTTGTGCGATATCGGCCACCTTTGCGATGCCAGCGGTAGCA
GATAATACCAAGCTGGGTGAAGAGCCAACCACCACCTTAAAGGGTGTATTGGTAAGCTCG
CAAACGAACCAAAATACAGGTTTTGTATCTAATGATTCAAAACAATCCAGTGATCTTACG
CTTTCAAAAGATAAATTAAAATATCGTTCGGCAACCTTGGGCAATGCGTTAAGTGGTGAG
CTTGGTATTCATAGTAACCCTTTTGGTGGCGGTTCATCTGCACCTGTTGTGCGAGGGCAA
GAGGGTGTGCGTCTTAAGATTTTACAAAATGGAACTGATGTGATTGATGTGTCATCAATA
TCGCCTGATCATGTTGTGGCGACCGATACACTTTTAGCGTCTAAAGTTGAGCTTGTTCGT
GGTGCTGATACGCTGTTATATGGCTTGGCATCGCCAGCTGGTGTGATTAATGTTGTTGAT
GACCGTATCCCGAATCGTATGCCTAGTGGTGCTATCCATGACAAAATCGAAGGCGAGACG
ATGCTTCGATATAACACAAACAACCATGAAAAGCTTGCAACTGCAGGGGTGAGCTTTGGG
GTAGGAGATCGCATTGCGGTTCGGGTGGAGGGCTTAAAGCGAGAGGCTGATGACTATCAA
GTTCCCCATTTTCAGGCAGATCGCATGTTAGATTATGTGCCAGGTAGTGCAAATAACTCT
ACCGTTGGCATGATTGGCGTGTCTTATATTCATGATAATGGGCATATCGGTGCTTCTTAT
AGCCACCGTAAAGATCGTTATGGTATCCCAGGGCATATCCACTGCGACAGCCAACGAGAG
CATTTTATCAAATGGCATAATATCACAAAATCCAATTATTATTTACCCATTTATCCTCAT
TTGATGGAGGATTCAGATATTGATGACAATCCTCATACGCATTGCCGCCACAACCACGAA
GACCATATCGGTGAGCATAATCCCACGGGCGTGCCCATCAATCATGAACATCATTCGCCT
TGGATTGATATGAAAACCAATCGCTACGACATTCGTGGCGAGGTATATCGGCCTATTCAA
GGTTTGGATAAAATTAAGCTAAGCTTAACTTATGCAGATTATTATCATGATGAAAAAGAT

GCTGGCAATGAGCAAGACCCAAACAATCACAAACCTTCTGAGCGTGATACAACGGTGGAT
AAGGGTCATGCCAGCTCTATTTTTACAAAAAAAGGCGTTAATGGTCGCTTGGAGTTATAT
CATACACCGACCAAACGCTTATCTGGGGTATTGGGTATTGGGTATCAAACCCAAAAATCT
GCAGCAGGAGAGGCGTATTTGCCAAGCTATTTTCAATCAGAAGCAGAATGGCAAAAAGCC
CAAAGTCAAAACATTAACCAATATCGTCCTTACTTATTAGTTCCAAATACCAATAAAAGC
CTTGGTATTTTTGGACTTGAGCAACTAAAGCTAAATCAAATGACTTTTAAGGTGGCGATG
CGTCATGAAAGACAAAAAACACCAATTGAATATGACCAGCATTTACTTGACCATGCTTTG
CAGTATTTTTTAAGTAAAGCACAGCTAAAAGCACCTGATCATCCTGATTTGACGACATAT
AAACAACATGCCACCTCTTATGCTGGTAGTGCCTTATGGGATATTACGCCAAATCATCGA
TTGTCATTGACCTACTCACATAACGAACGCATTCCATCGCCGATGGAGCTGTATTATCAA
GGCGGACATTTGGCGACCAGCTCTTTTGAGCATGGCAATAAAAACTTGGTCAAAGAAAAA
TCGGATAATTATGAGCTGGGTTTTATGCATACAGCAGATAAAGTCAGCTATAAAGCAAGC
ACTTACTATAGCAATTTTGATAACTATATTTTTAATGAGACCATTGCCAAAGAAGGCAAT
TTATACATCAGACGCTATAATCAGACGACGGCTAAGTTTTATGGTGTGGAGGGTTCATTA
ACTTACCAGCCAAATGCCAATCACAGTGTGATGTTTTTTGGTGATATGGTGCAAGGTAAA
ATTGGTGCATTATCTGATATTAAAGGTAAGCTTGTATATGCTGGCAGAAAGTGGGTTTAT
TTTGATGATGATATCAAGGATATGACCGTTGATGATAATGGTGATTATGATGCCGATGGT
GGTTTGACTTGTGCCTTAAAAACGCCTGAACAATGGGGACAAATTAACGATAATAATGAT
TGTAGTACAACCATTAATGTCTATAAAAACGGTACAACAACCTCAGGCGAGGAGGATTAC
GACCGTTTGGCACGAAATCCAACTTACGCACCCAGAGTACCGCCCAGCCGCTTGGGCATT
CGTTGGCAAGGGTATTTTGGTGATCATTGGTCTGCCAATGCAGAATTTAACCATGTGTTT
GCACAAAATAAAGTTGCCACCTCAACGGTTGCCATTAAACCTCAATTCAAGCAGCCAGAA
GGTTGCCAACGCCATGAGAGTCATTGCCGAATCAGCGACTATGGCAGTGATAACAACCCT
TTGATGATGCAGCCAAGATATATCACAGAAAACAAAACGGCAGGATATAATTTGCTCAAT
GTTGGCTTAGATTATAACAATGCATATCGTAATGTTGATTATACGCTGTCAATTCGTGCG
AATAATTTACTTAATGAACAAATCTACATTCACAACTCATTTTTGCCGTTTGTACCGCAG
ATGGGGCGTAATCTGACTTTAGGTTTGACGGCTAAATTTTGA

**SEQ ID NO: 12**

***Moraxella catarrhalis*** **BASB108 polypeptide sequence deduced from the polynucleotide of SEQ ID NO:11**

```
MIKKPLVCAISATFAMPAVADNTKLGEEPTTTLKGVLVSSQTNQNTGFVSNDSKQSSDLTLSKDKLKYRSATLGNALSGELGIHSNPF
GGGSSAPVVRGQEGVRLKILQNGTDVIDVSSISPDHVVATDTLLASKVELVRGADTLLYGLASPAGVINVVDDRIPNRMPSGAIHDKI
EGETMLRYNTNNHEKLATAGVSFGVGDRIAVRVEGLKREADDYQVPHFQADRMLDYVPGSANNSTVGMIGVSYIHDNGHIGASYSHRK
DRYGIPGHIHCDSQREHFIKWHNITKSNYYLPIYPHLMEDSDIDDNPHTHCRHNHEDHIGEHNPTGVPINHEHHSPWIDMKTNRYDIR
GEVYRPIQGLDKIKLSLTYADYYHDEKDAGNEQDPNNHKPSERDTTVDKGHASSIFTKKGVNGRLELYHTPTKRLSGVLGIGYQTQKS
AAGEAYLPSYFQSEAEWQKAQSQNINQYRPYLLVPNTNKSLGIFGLEQLKLNQMTFKVAMRHERQKTPIEYDQHLLDHALQYFLSKAQ
LKAPDHPDLTTYKQHATSYAGSALWDITPNHRLSLTYSHNERIPSPMELYYQGGHLATSSFEHGNKNLVKEKSDNYELGFMHTADKVS
YKASTYYSNFDNYIFNETIAKEGNLYIRRYNQTTAKFYGVEGSLTYQPNANHSVMFFGDMVQGKIGALSDIKGKLVYAGRKWVYFDDD
IKDMTVDDNGDYDADGGLTCALKTPEQWGQINDNNDCSTTINVYKNGTTTSGEEDYDRLARNPTYAPRVPPSRLGIRWQGYFGDHWSA
NAEFNHVFAQNKVATSTVAIKPQFKQPEGCQRHESHCRISDYGSDNNPLMMQPRYITENKTAGYNLLNVGLDYNNAYRNVDYTLSIRA
NNLLNEQIYIHNSFLPFVPQMGRNLTLGLTAKF
```

## Claims

1. An isolated polypeptide comprising an amino acid sequence which has at least 85% identity to the amino acid sequence of SEQ ID NO: 2.

2. The polypeptide as claimed in claim 1 in which the amino acid sequence has at least 95% identity to the amino acid sequence of SEQ ID NO: 2.

3. The polypeptide as claimed in claim 1 comprising the amino acid sequence of SEQ ID NO: 2.

4. An isolated polypeptide of SEQ ID NO: 2.

5. An immunogenic fragment of the polypeptide as claimed in any one of claims 1 to 4 in which said immunogenic fragment, if necessary when coupled to a carrier, is capable of raising an immune response which recognises the polypeptide of SEQ ID NO: 2, with the proviso that said fragment does not have the sequence nvvtntgatv vdgtrtifst lvkpaawaa v.

6. A fusion protein comprising a polypeptide or an immunogenic fragment as claimed in any one of claims 1 to 5 with the proviso that said fusion protein does not have the sequence nvvtntgatv vdgtrtifst lvkpaawaa v.

7. An isolated polynucleotide comprising a nucleotide sequence encoding a polypeptide that has at least 85% identity to the amino acid sequence of SEQ ID NO: 2, over the entire length of SEQ ID NO: 2; or a nucleotide sequence complementary to said isolated polynucleotide.

8. An isolated polynucleotide comprising a nucleotide sequence that has at least 85% identity to a nucleotide sequence encoding a polypeptide of SEQ ID NO: 2, over the entire coding region; or a nucleotide sequence complementary to said isolated polynucleotide.

9. An isolated polynucleotide which comprises a nucleotide sequence which has at least 85% identity to that of SEQ ID NO: 1, over the entire length of SEQ ID NO: 1; or a nucleotide sequence complementary to said isolated polynucleotide.

10. The isolated polynucleotide as claimed in any one of claims 7 to 9 in which the identity is at least 95% to SEQ ID NO:1.

11. An isolated polynucleotide comprising a nucleotide sequence encoding the polypeptide, the immunogenic fragment or the fusion protein of any one of claims 1 to 6.

12. An isolated polynucleotide comprising the polynucleotide of SEQ ID NO: 1.

**13.** An isolated polynucleotide comprising a nucleotide sequence encoding the polypeptide of SEQ ID NO: 2 obtainable by screening an appropriate library under stringent hybridization conditions with a labelled probe having the sequence of SEQ ID NO: 1.

**14.** An expression vector, expression system or a recombinant live microorganism comprising an isolated, recombinant polynucleotide according to any one of claims 7 to 13.

**15.** A host cell comprising the expression vector of claim 14 or a subcellular fraction or a membrane of said host cell expressing an isolated polypeptide, an immunogenic fragment or a fusion protein as claimed in any one of claims 1 to 6.

**16.** A process for producing a polypeptide, an immunogenic fragment or fusion protein of any one of claims 1 to 6 comprising culturing a host cell of claim 15 under conditions sufficient for the production of said polypeptide, said immunogenic fragment or said fusion protein and recovering the polypeptide, the immunogenic fragment or the fusion protein from the culture medium.

**17.** A process for expressing a polynucleotide of any one of claims 7 to 13 comprising transforming a host cell with the expression vector of claim 14 and culturing said host cell under conditions sufficient for expression of any one of said polynucleotides.

**18.** A vaccine composition comprising an effective amount of the polypeptide or the immunogenic fragment of any one of claims 1 to 6 and a pharmaceutically acceptable carrier.

**19.** A vaccine composition comprising an effective amount of the polynucleotide of any one of claims 7 to 13 and a pharmaceutically acceptable carrier.

**20.** The vaccine composition according to either one of claims 18 or 19 wherein said composition comprises at least one other *Moraxella catarrhalis* antigen.

**21.** An antibody immunospecific for the polypeptide or immunogenic fragment as claimed in any one of claims 1 to 5.

**22.** A method of diagnosing a *Moraxella catarrhalis* infection, comprising identifying a polypeptide or an immunogenic fragment as claimed in any one of claims 1 to 5, or an antibody of claim 21, present within a biological sample from an animal suspected of having such an infection.

**23.** Use of a composition comprising an immunologically effective amount of a polypeptide or an immunogenic fragment as claimed in any one of claims 1 to 6 in the preparation of a medicament for use in generating an immune response in an animal.

**24.** Use of a composition comprising an immunologically effective amount of a polynucleotide as claimed in any one of claims 7 to 13 in the preparation of a medicament for use in generating an immune response in an animal.

**25.** A therapeutic composition useful in treating humans with *Moroxella catarrhalis* disease comprising at least one antibody as claimed in claim 21 and a suitable pharmaceutical carrier.

**Patentansprüche**

**1.** Isoliertes Polypeptid, das eine Aminosäuresequenz umfasst, die wenigstens 85 % Identität zu der Aminosäuresequenz von SEQ ID NO: 2 aufweist.

**2.** Polypeptid gemäss Anspruch 1, worin die Aminosäuresequenz wenigstens 95 % Identität zu der Aminosäuresequenz von SEQ ID NO: 2 aufweist.

**3.** Polypeptid gemäss Anspruch 1, das die Aminosäuresequenz von SEQ ID NO: 2 umfasst.

**4.** Isoliertes Polypeptid von SEQ ID NO: 2.

5. Immunogenes Fragment des Polypeptids gemäß einem der Ansprüche 1 bis 4, worin das immunogene Fragment, falls erforderlich bei Kopplung an einen Träger, in der Lage ist, eine Immunreaktion hervorzurufen, die das Polypeptid von SEQ ID NO: 2 erkennt, mit der Maßgabe, dass das Fragment nicht die Sequenz nvvtntgatv vdgtrtifst lvkpaavvaa v aufweist.

6. Fusionsprotein, das ein Polypeptid oder ein immunogenes Fragment gemäß einem der Ansprüche 1 bis 5 umfasst, mit der Maßgabe, dass das Fusionsprotein nicht die Sequenz nvvtntgatv vdgtrtifst lvkpaavvaa v aufweist.

7. Isoliertes Polynukleotid, das eine Nukleotidsequenz umfasst, die ein Polypeptid codiert, das wenigstens 85 % Identität zu der Aminosäuresequenz von SEQ ID NO: 2 über die gesamte Länge von SEQ ID NO: 2 aufweist; oder Nukleotidsequenz, die zu dem isolierten Polynukleotid komplementär ist.

8. Isoliertes Polynukleotid, das eine Nukleotidsequenz umfasst, die wenigstens 85 % Identität zu einer Nukleotidsequenz, die ein Polypeptid von SEQ ID NO: 2 codiert, über die gesamte codierende Region aufweist; oder Nukleotidsequenz, die zu dem isolierten Polynukleotid komplementär ist.

9. Isoliertes Polynukleotid, das eine Nukleotidsequenz umfasst, die wenigstens 85 % Identität zu der von SEQ ID NO: 1 über die gesamte Länge von SEQ ID NO: 1 aufweist; oder Nukleotidsequenz, die zu dem isolierten Polynukleotid komplementär ist.

10. Isoliertes Polynukleotid gemäß einem der Ansprüche 7 bis 9, worin die Identität wenigstens 95 % zu SEQ ID NO: 1 ist.

11. Isoliertes Polynukleotid, das eine Nukleotidsequenz umfasst, die das Polypeptid, das immunogene Fragment oder das Fusionsprotein gemäß einem der Ansprüche 1 bis 6 codiert.

12. Isoliertes Polynukleotid, das das Polynukleotid von SEQ ID NO: 1 umfasst.

13. Isoliertes Polynukleotid, das eine Nukleotidsequenz umfasst, die das Polypeptid von SEQ ID NO: 2 codiert, erhältlich durch Durchmustern einer entsprechenden Bibliothek unter stringenten Hybridisierungsbedingungen mit einer markierten Sonde, die die Sequenz von SEQ ID NO: 1 aufweist.

14. Expressionsvektor, Expressionssystem oder ein rekombinanter, lebender Mikroorganismus, der/das ein isoliertes, rekombinantes Polynukleotid gemäß einem der Ansprüche 7 bis 13 umfasst.

15. Wirtszelle, die den Expressionsvektor von Anspruch 14 umfasst, oder eine subzelluläre Fraktion oder eine Membran der Wirtszelle, die ein isoliertes Polypeptid, ein immunogenes Fragment oder ein Fusionsprotein gemäß einem der Ansprüche 1 bis 6 exprimiert.

16. Verfahren zur Herstellung eines Polypeptids, eines immunogenen Fragments oder eines Fusionsproteins gemäß einem der Ansprüche 1 bis 6, das das Kultivieren einer Wirtszelle von Anspruch 15 unter Bedingungen, die für die Herstellung des Polypeptids, des immunogenen Fragments oder des Fusionsproteins genügend sind, und das Gewinnen des Polypeptids, des immunogenen Fragments oder des Fusionsproteins aus dem Kulturmedium umfasst.

17. Verfahren zur Expression eines Polynukleotids gemäß einem der Ansprüche 7 bis 13, das das Transformieren einer Wirtszelle mit dem Expressionsvektor von Anspruch 14 und das Kultivieren der Wirtszelle unter Bedingungen umfasst, die für die Expression irgendeines der Polynukleotide genügend sind.

18. Impfstoffzusammensetzung, die eine effektive Menge des Polypeptids oder des immunogenen Fragments gemäß einem der Ansprüche 1 bis 6 und einen pharmazeutisch annehmbaren Träger umfasst.

19. Impfstoffzusammensetzung, die eine effektive Menge des Polynukleotids gemäß einem der Ansprüche 7 bis 13 und einen pharmazeutisch annehmbaren Träger umfasst.

20. Impfstoffzusammensetzung gemäß Anspruch 18 oder 19, worin die Zusammensetzung wenigstens ein anderes Antigen von Moraxella catarrhalis umfasst.

21. Antikörper, der für das Polypeptid oder immunogene Fragment gemäß einem der Ansprüche 1 bis 5 immunspezifisch ist.

**22.** Verfahren zur Diagnose einer Infektion mit Moraxella catarrhalis, das das Identifizieren eines Polypeptids oder eines immunogenen Fragments gemäß einem der Ansprüche 1 bis 5 oder eines Antikörpers gemäß Anspruch 21, das/der in einer biologischen Probe aus einem Tier vorliegt, von dem vermutet wird, dass es eine solche Infektion aufweist, umfasst.

**23.** Verwendung einer Zusammensetzung, die eine immunologisch effektive Menge eines Polypeptids oder eines immunogenen Fragments gemäß einem der Ansprüche 1 bis 6 umfasst, in der Herstellung eines Medikaments zur Verwendung in der Erzeugung einer Immunreaktion in einem Tier.

**24.** Verwendung einer Zusammensetzung, die eine immunologisch effektive Menge eines Polynukleotids gemäß einem der Ansprüche 7 bis 13 umfasst, in der Herstellung eines Medikaments für die Verwendung in der Erzeugung einer Immunreaktion in einem Tier.

**25.** Therapeutische Zusammensetzung, die in der Behandlung von Menschen mit einer Moraxella catarrhalis-Erkrankung nützlich ist, umfassend wenigstens einen Antikörper gemäß Anspruch 21 und einen geeigneten pharmazeutischen Träger.

## Revendications

**1.** Polypeptide isolé comprenant une séquence d'acides aminés au moins identique à 85 % à la séquence d'acides aminés de SEQ ID NO: 2.

**2.** Polypeptide selon la revendication 1, dans lequel la séquence d'acides aminés est au moins identique à 95 % à la séquence d'acides aminés de SEQ ID NO: 2.

**3.** Polypeptide selon la revendication 1 comprenant la séquence d'acides aminés de SEQ ID NO: 2.

**4.** Polypeptide isolé de SEQ ID NO: 2.

**5.** Fragment immunogène du polypeptide selon l'une quelconque des revendications 1 à 4, dans lequel ledit fragment immunogène, si nécessaire lorsqu'il est couplé à un support, est capable de déclencher une réaction immunitaire qui reconnaît le polypeptide de SEQ ID NO: 2, à condition que ledit fragment n'ait pas la séquence « nvvtntgatv vdgtrtifst lvkpaavvaa v ».

**6.** Protéine de fusion comprenant un polypeptide ou un fragment immunogène selon l'une quelconque des revendications 1 à 5, à condition que ladite protéine de fusion n'ait pas la séquence « nvvtntgatv vdgtrtifst lvkpaavvaa v ».

**7.** Polynucléotide isolé comprenant une séquence de nucléotides codant pour un polypeptide qui est au moins identique à 85 % à la séquence d'acides aminés de SEQ ID NO: 2, sur toute la longueur de SEQ ID NO: 2, ou une séquence de nucléotides complémentaire audit polynucléotide isolé.

**8.** Polynucléotide isolé comprenant une séquence de nucléotides qui est au moins identique à 85 % à une séquence de nucléotides codant pour un polypeptide de SEQ ID NO: 2, sur toute la région codante ; ou une séquence de nucléotides complémentaire audit polynucléotide isolé.

**9.** Polynucléotide isolé qui comprend une séquence de nucléotides qui est au moins identique à 85 % à celle de SEQ ID NO:1 ; ou toute la longueur de SEQ ID NO: 1 ; ou la séquence de nucléotides complémentaire audit polynucléotide isolé.

**10.** Polynucléotide isolé selon l'une quelconque des revendications 7 à 9, dans laquelle l'identité est d'au moins de 95 % avec SEQ ID NO: 1.

**11.** Polynucléotide isolé comprenant une séquence de nucléotides codant pour le polypeptide, le fragment immunogène ou la protéine de fusion selon l'une quelconque des revendications 1 à 6.

**12.** Polynucléotide isolé comprenant le polynucléotide de SEQ ID NO:1.

13. Polynucléotide isolé comprenant une séquence de nucléotides codant pour le polypeptide de SEQ ID NO: 2 qui peut être obtenu en criblant une collection appropriée dans des conditions d'hybridation stringentes avec une sonde marquée ayant la séquence de SEQ ID NO: 1.

14. Vecteur d'expression, système d'expression ou microorganisme vivant recombinant comprenant un polynucléotide recombinant isolé selon l'une quelconque des revendications 7 à 13.

15. Cellule hôte comprenant le vecteur d'expression selon la revendication 14 ou une fraction sous-cellulaire ou une membrane de ladite cellule hôte exprimant un polynucléotide isolé, un fragment immunogène ou une protéine de fusion selon l'une quelconque des revendications 1 à 6.

16. Procédé de production d'un polypeptide, d'un fragment immunogène ou d'une protéine de fusion selon l'une quelconque des revendications 1 à 6, comprenant les étapes consistant à cultiver une cellule hôte selon la revendication 15 dans des conditions suffisantes pour la production dudit polypeptide, dudit fragment immunogène ou de ladite protéine de fusion, et à récupérer le polypeptide, le fragment immunogène ou la protéine de fusion à partir du milieu de culture.

17. Procédé d'expression d'un polynucléotide selon l'une quelconque des revendications 7 à 13, comprenant les étapes consistant à transformer une cellule hôte avec le vecteur d'expression selon la revendication 14, et à cultiver ladite cellule hôte dans des conditions suffisantes pour l'expression de l'un quelconque desdits polynucléotides.

18. Composition de vaccin comprenant une quantité efficace du polypeptide ou du fragment immunogène selon l'une quelconque des revendications 1 à 6 et un support acceptable sur le plan pharmaceutique.

19. Composition de vaccin comprenant une quantité efficace du polynucléotide selon l'une quelconque des revendications 7 à 13 et un support acceptable sur le plan pharmaceutique.

20. Composition de vaccin selon l'une des revendications 18 ou 19, dans laquelle ladite composition comprend au moins un autre antigène de *Moraxella catarrhalis.*

21. Anticorps immuno-spécifique pour le polypeptide ou le fragment immunogène selon l'une quelconque des revendications 1 à 5.

22. Procédé de diagnostic d'une infection par *Moraxella catarrhalis,* comprenant l'identification d'un polypeptide ou d'un fragment immunogène selon l'une quelconque des revendications 1 à 5, ou un anticorps selon la revendication 21, présent dans un échantillon biologique prélevé chez un animal suspecté d'être porteur de cette infection.

23. Utilisation d'une composition comprenant une quantité efficace du point de vue immunologique d'un polypeptide ou d'un fragment immunogène selon l'une quelconque des revendications 1 à 6, dans la préparation d'un médicament utilisable pour générer une réaction immunitaire chez un animal.

24. Utilisation d'une composition comprenant une quantité efficace du point de vue immunologique d'un polynucléotide selon l'une quelconque des revendications 7 à 13, dans la préparation d'un médicament utilisable pour générer une réaction immunitaire chez un animal.

25. Composition thérapeutique utilisable pour traiter les humains atteints de la maladie liée à *Moraxella catarrhalis,* comprenant au moins un anticorps selon la revendication 21 et un support adapté du point de vue pharmaceutique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9429458 A
- WO 9422914 A
- US 4946778 A
- EP 0552267 B1
- WO 9400153 A
- WO 9517209 A
- GB 2220211 A, Ribi
- EP 0689454 B1
- EP 0689454 A
- US 5057540 A
- WO 9633739 A
- WO 9602555 A
- WO 9517210 A


### Non-patent literature cited in the description


- **KLEIN, JO.** *Clin.Inf.Dis,* 1994, vol. 19, 823
- **MURPHY, TF.** *Microbiol.Rev.,* 1996, vol. 60, 267
- **DICKINSON, DP et al.** *J. Infect.Dis.,* 1988, vol. 158, 205
- **FADEN, HL et al.** *Ann.Otorhinol.Laryngol.,* 1991, vol. 100, 612
- **FADEN, HL et al.** *J. Infect.Dis.,* 1994, vol. 169, 1312
- **CHAPMAN, AJ et al.** *J. Infect.Dis.,* 1985, vol. 151, 878
- **HOL, C et al.** *Lancet,* 1993, vol. 341, 1381
- **JORDAN, KL et al.** *Am.J.Med.,* 1990, vol. 88 (5A), 28S
- **SETHI, S et al.** *Infect.Immun.,* 1995, vol. 63, 1516
- **CHEN D. et al.** *Infect.Immun.,* 1999, vol. 67, 1310
- *Gene,* 1986, vol. 43, 265-272
- *Biotechnology,* 1992, vol. 10, 795-798
- **MANIATIS, T. ; FRITSCH, E.F. ; SAMBROOK et al.** MOLECULAR CLONING, A LABORATORYMANUAL. Cold Spring Harbor Laboratory Press, 1989
- **GENTZ et al.** *Proc. Natl. Acad. Sci., USA,* 1989, vol. 86, 821-824
- **WILSON et al.** *Cell,* 1984, vol. 37, 767
- **SAMBROOK et al.** Molecular Cloning: A Laboratory Manual. Cold Spring Harbor, 1989
- **FROHMAN et al.** *PNAS USA,* 1988, vol. 85, 8998-9002
- **WOLFF et al.** *Hum Mol Genet,* 1992, vol. 1, 363
- **MANTHORPE et al.** *Hum. Gene Ther.,* 1983, vol. 4, 419
- **WU et al.** *J Biol Chem.,* 1989, vol. 264, 16985
- **BENVENISTY ; RESHEF.** *PNAS USA,* 1986, vol. 83, 9551
- **KANEDA et al.** *Science,* 1989, vol. 243, 375
- **TANG et al.** *Nature,* 1992, vol. 356, 152
- **EISENBRAUN et al.** *DNA Cell Biol,* 1993, vol. 12, 791
- **SEEGER et al.** *PNAS USA,* 1984, vol. 81, 5849
- **DAVIS et al.** *BASIC METHODS IN MOLECULAR BIOLOGY,* 1986
- **SAMBROOK et al.** MOLECULAR CLONING: A LABORATORY MANUAL. Cold Spring Harbor Laboratory Press, 1989
- **SAMBROOK et al.** *MOLECULAR CLONING, A LABORATORY MANUAL*
- **MYERS et al.** *Science,* 1985, vol. 230, 1242
- **COTTON et al.** *Proc. Natl. Acad. Sci., USA,* 1985, vol. 85, 4397-4401
- **CHEE et al.** *Science,* 1996, vol. 274, 610
- **KOHLER, G. ; MILSTEIN, C.** *Nature,* 1975, vol. 256, 495-497
- **KOZBOR et al.** *Immunology Today,* 1983, vol. 4, 72
- **COLE et al.** MONOCLONAL ANTIBODIES AND CANCER THERAPY. Alan R. Liss; Inc, 1985, 77-96
- **MCCAFFERTY et al.** *Nature,* 1990, vol. 348, 552-554
- **MARKS et al.** *Biotechnologv,* 1992, vol. 10, 779-783
- **CLACKSON et al.** *Nature,* 1991, vol. 352, 628
- **JONES et al.** *Nature,* 1986, vol. 321, 522-525
- **TEMPEST et al.** *Biotechnology,* 1991, vol. 9, 266-273
- **COLIGAN et al.** Current Protocols in Immunology. 1991, vol. 1
- **D. BENNETT et al.** *J Mol Recognition,* 1995, vol. 8, 52-58
- **K. JOHANSON et al.** *J Biol Chem,* 1995, vol. 270 (16), 9459-9471

- **OKANO.** *J. Neurochem.,* 1991, vol. 56, 560
- OLIGODEOXYNUCLEOTIDES AS ANTISENSE INHIBITORS OF GENE EXPRESSION. CRC Press, 1988
- **SATO, Y. et al.** *Science,* 1996, vol. 273, 352
- **MOSMANN, T.R. ; COFFMAN, R.L.** TH1 and TH2 cells: different patterns of lymphokine secretion lead to different functional properties. *Annual Review of Immunology,* 1989, vol. 7, 145-173
- Computational Molecular Biology. Oxford University Press, 1988
- Biocomputing: Informatics and Genome Projects. Academic Press, 1993
- Computer Analysis of Sequence Data. Humana Press, 1994
- **HEINE, G.** Sequence Analysis in Molecular Biology. Academic Press, 1987
- Sequence Analysis Primer. M Stockton Press, 1991
- **CARILLO, H. ; LIPMAN, D. ; SIAM J.** *Applied Math.,* 1988, vol. 48, 1073
- **DEVEREUX, J. et al.** *Nucleic Acids Research,* 1984, vol. 12 (1), 387
- **ALTSCHUL, S.F. et al.** *J Molec. Biol.,* 1990, vol. 215, 403-410
- **PEARSON ; LIPMAN.** *Proc. Natl. Acad. Sci. USA,* 1988, vol. 85, 2444-2448
- **ALTSCHUL, S. et al.** *J Mol. Biol.,* 1990, vol. 215, 403-410
- **NEEDLEMAN ; WUNSCH.** *J. Mol Biol.,* 1970, vol. 48, 443-453
- **HENIKOFF ; HENIKOFF.** *Proc. Natl. Acad. Sci. USA.,* 1992, vol. 89, 10915-10919